(19) Europäisches Patentamt
European Patent Office
Office européen des brevets

(11) Veröffentlichungsnummer: **0 173 322**
**B1**

(12) # EUROPÄISCHE PATENTSCHRIFT

(45) Veröffentlichungstag der Patentschrift: **28.12.88**

(51) Int. Cl.[4]: **C 07 D 239/42**, C 07 D 239/46, C 07 D 213/75, A 01 N 47/36

(21) Anmeldenummer: **85110836.5**

(22) Anmeldetag: **19.08.85**

(54) **(1-(2-Oxyaminosulfonylphenylsulfonyl)-3-heteroaryl-harnstoffe.**

(30) Priorität: **30.08.84 DE 3431929**
**25.05.85 DE 3518876**

(43) Veröffentlichungstag der Anmeldung:
**05.03.86 Patentblatt 86/10**

(45) Bekanntmachung des Hinweises auf die Patenterteilung:
**28.12.88 Patentblatt 88/52**

(84) Benannte Vertragsstaaten:
**AT BE CH DE FR GB IT LI NL SE**

(56) Entgegenhaltungen:
**EP-A- 0 048 143**
**EP-A- 0 074 282**
**EP-A- 0 085 028**
**EP-A- 0 102 925**
**EP-A- 0 116 518**
**EP-A- 0 128 274**
**US-A- 4 372 778**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Koeln 80 (DE)**
Erfinder: **Schmidt, Robert R., Dr., Im Waldwinkel 110, D-5060 Bergisch-Gladbach 2 (DE)**

**EP 0 173 322 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 1-(2-Oxyaminosulfonylphenyl-sulfonyl)-3-heteroaryl-harnstoffe, Verfahren zu ihrer Herstellung und ihre Verwendung als Herbizide.

Es ist bekannt, dass bestimmte 1-Arylsulfonyl-3-heteroaryl-harnstoffe, wie z.B. 1-(2-Methoxyphenylsulfonyl)-3-(4,6-dimethyl-pyrimidin-2-yl)-harnstoff, herbizid wirksam sind. Die Wirkung dieser Verbindungen ist jedoch nicht immer ganz befriedigend (vergl. US-PS 4 169 719).

Es wurden nun neue 1-(2-Oxyaminosulfonylphenylsulfonyl)-3-heteroaryl-harnstoffe der allgemeinen Formel (I)

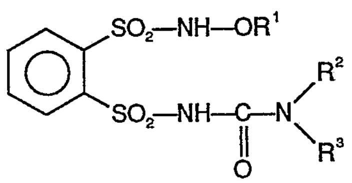
(I)

in welcher
$R^1$ für $C_1$-$C_{12}$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl, oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht, in welcher weiter
für Wasserstoff, $C_1$-$C_4$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl,$C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl, $C_3$-$C_6$-Alkinyl oder Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder$C_1$-$C_4$-Alkoxy-carbonyl substituiert ist) steht, in welcher ferner
$R^3$ für den Rest

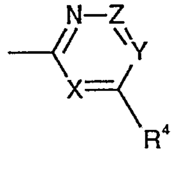

steht, worin
$R^4$ für Wasserstoff, Fluor Chlor, Brom, Hydroxy, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio (welches gegebenenfalls durch Fluor und/oder Chlor substituiert ist), Amino, $C_1$-$C_4$-Alkylamino oder Di-($C_1$-$C_4$-alkyl)-amino steht,
X für Stickstoff oder eine Methin-brücke (CH) steht,
Y für Stickstoff oder eine gegebenenfalls substituierte Methin-brücke C-$R^5$ steht, wobei
$R^5$ für Wasserstoff, Fluor, Chlor, Brom, Cyano, Formyl, $C_1$-$C_4$-Alkyl, $C_1$-$C_3$-Alkoxy-carbonyl oder $C_1$-$C_3$-Alkyl-carbonyl steht, und
Z für Stickstoff oder eine gegebenenfalls substituierte Methin-brücke C-$R^6$ steht, wobei,
$R^6$ für Wasserstoff, Fluor, Chlor, Brom, Hydroxy, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio (welches gegebenenfalls durch Fluor und/oder Chlor substituiert ist), Amino, $C_1$-$C_4$-Alkylamino oder Di($C_1$-$C_4$-alkyl)-amino steht, gefunden,
wobei folgende Verbindungen ausgenommen sind:

1-(2-Methoxyaminosulfonylphenylsulfonyl)-,
1-(2-Ethoxyaminosulfonylphenylsulfonyl)-,
1-(2-Propoxyaminosulfonylphenylsulfonyl)-,
1-(2-Isopropoxyaminosulfonylphenylsulfonyl)- und
1-(2-Butoxyaminosulfonylphenylsulfonyl)-,
-3-(4,6-dimethyl-pyrimidin-2-yl)-harnstoff,
-3-(4,6-diethyl-pyrimidin-2-yl)-harnstoff,
-3-(4,6-dipropyl-pyrimidin-2-yl)-harnstoff,
-3-(4,6-diisopropyl-pyrimidin-2-yl)-harnstoff und
-3-(4,6-dibutyl-pyrimidin-2-yl)-harnstoff.

Man erhält die neuen Verbindungen der Formel (I), wenn man
(a) Benzodisultame der Formel (II)

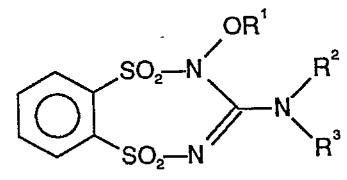
(II)

in welcher
$R^1$, $R^2$ und $R^3$ die oben angegebenen Bedeutungen haben,
mit Wasser gegebenenfalls in Gegenwart von Basen und gegebenenfalls in Gegenwart von Verdünnungsmitteln umsetzt, oder
(b) Benzol-1,2-disulfonsäure-dichlorid der Formel (III)

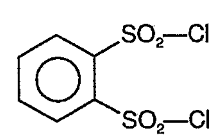
(III)

mit Oxyguanidin-Derivaten der Formel (IV)

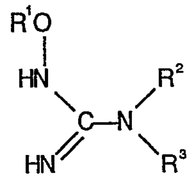

(IV)

in welcher
$R^1$, $R^2$ und $R^3$ die oben angegebene Bedeutung haben, in Gegenwart von Säureakzeptoren und gegebenenfalls in Gegenwart von Verdünnungsmitteln umsetzt und die hierbei erhaltenen Verbindungen der Formel (II) - ohne Zwischenisolierung - mit Wasser gegebenenfalls in Gegenwart von Basen und gegebenenfalls in Gegenwart von Verdünnungsmitteln umsetzt.

Die neuen 1-(2-Oxyaminosulfonylphenylsulfonyl)-3-heteroaryl-harnstoffe der Formel (I) zeichnen sich durch starke herbizide Wirksamkeit aus.

Überraschenderweise zeigen die neuen Verbindungen der Formel (I) erheblich bessere herbizide Wirkung als vorbekannte Harnstoff-Derivate gleicher Wirkungsrichtung.

Gegenstand der Erfindung sind vorzugsweise Verbindungen der Formel (I) in welcher
$R^1$ für $C_1$-$C_8$-Alkyl (welches gegebenenfalls durch Fluor oder Chlor substituiert ist), $C_3$-$C_4$-Alkenyl, $C_1$-$C_2$-Alkoxy-carbonylmethyl, Phenyl, Phenylethyl oder Benzyl (welches gegebenenfalls durch Fluor, Chlor, Nitro, Cyano, Methyl, Methoxy oder Methoxy-carbonyl substituiert ist) steht,
$R^2$ für Wasserstoff steht und
$R^3$ für den Rest

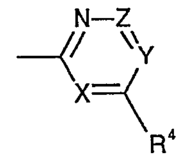

steht, worin
$R^4$ für Chlor, Methyl, Ethyl, Methoxy, Difluormethoxy oder Ethoxy steht,
X für Stickstoff steht,
Y für eine Methin-brücke (CH) steht, und
Z für eine gegebenenfalls substituierte Methinbrücke C-$R^6$ steht, wobei
$R^6$ für Wasserstoff, Chlor, Methyl, Methoxy, Ethoxy, Methylthio, Ethylthio, Dimethylamino oder Diethylamino steht,

wobei die oben wörtlich genannten Verbindungen ausgenommen sind.

Die bei dem oben unter (a) angegebenen erfindungsgemässen Herstellungsverfahren ablaufende chemische Reaktion kann beispielsweise durch folgendes Formelschema skizziert werden:

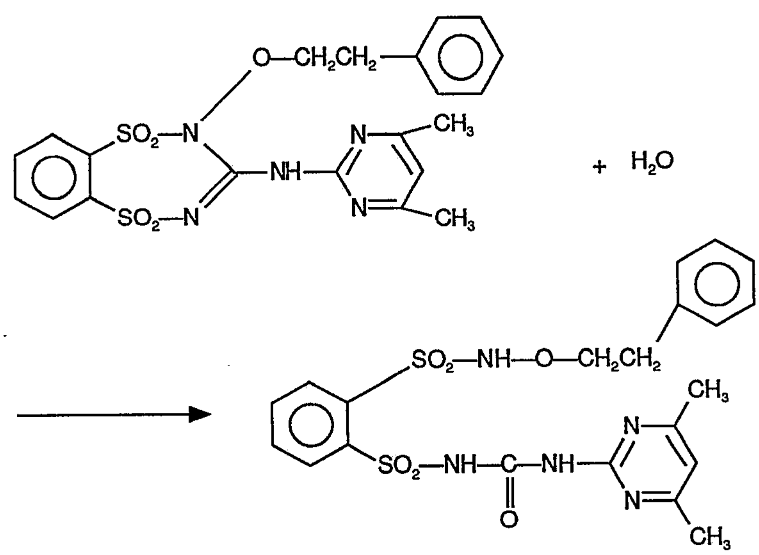

Die bei dem oben unter (b) angegebenen erfindungsgemässen Herstellungsverfahren ablaufenden Reaktionen können beispielsweise durch folgendes Formelschema skizziert werden:

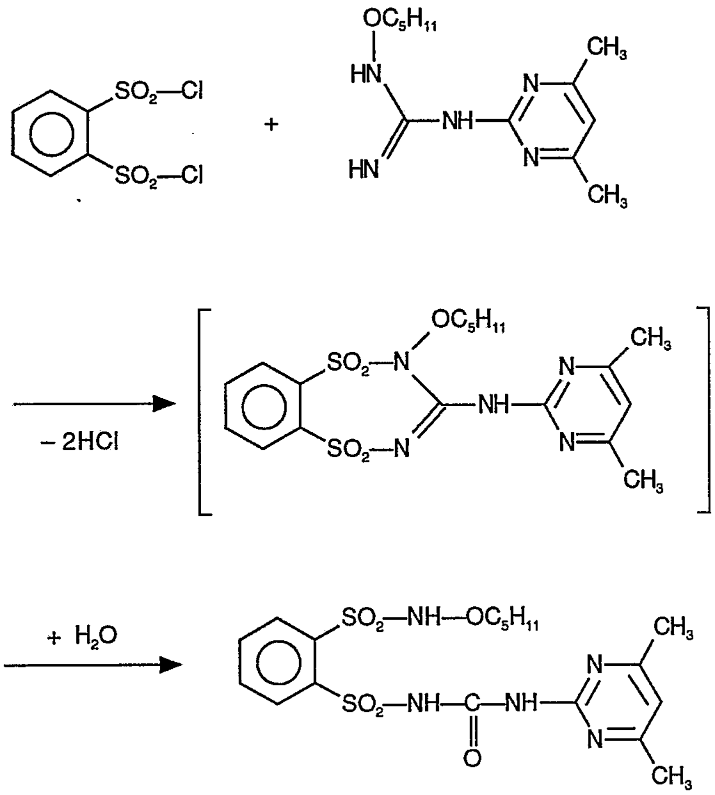


Die beim erfindungsgemässen Verfahren (a) als Ausgangsstoffe zu verwendenden Benzodisultame sind durch die Formel (II) allgemein definiert.

In Formel (II) haben $R^1$, $R^2$ und $R^3$ vorzugsweise die gleichen Bedeutungen, wie sie oben im Rahmen der Substituentendefinition der Formel (I) vorzugsweise angegeben sind.

Beispiele für die Ausgangsstoffe der Formel (II) sind in der nachstehenden Tabelle 1 aufgeführt.

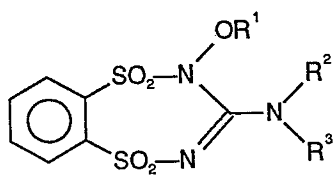

(II)

Tabelle 1

Beispiele für Ausgangsstoffe der Formel (II)

| $R^1$ | $R^2$ | $R^3$ |
|---|---|---|
| 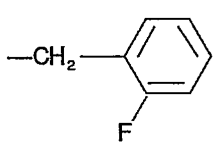 | H | 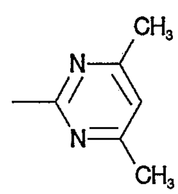 |
| $—CH_3$ | $—CH_3$ | 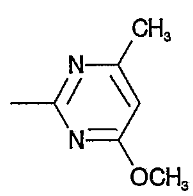 |

Tabelle 1 (Fortsetzung)

| $R^1$ | $R^2$ | $R^3$ |
|---|---|---|
| 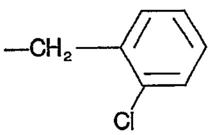 | H | 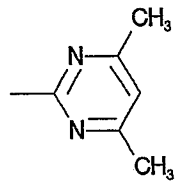 |
| $-C_3H_7$ | H | 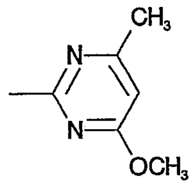 |
| $-CH(CH_3)_2$ | H | 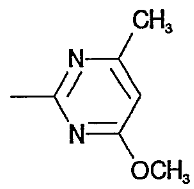 |
| $-C_4H_9$ | H | 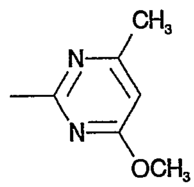 |
| $-CH_2CH(CH_3)_2$ | H | 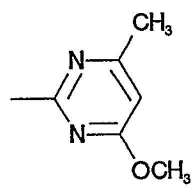 |
| $-C_8H_{17}$ | H | 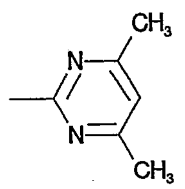 |
| 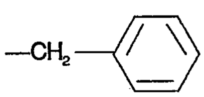 | H | 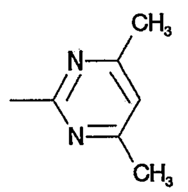 |
| 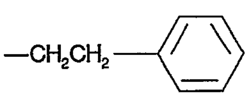 | H | 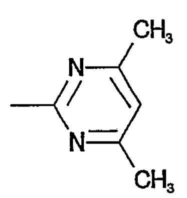 |

Tabelle 1 (Fortsetzung)

| $R^1$ | $R^2$ | $R^3$ |
|---|---|---|
| 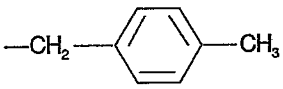 | H | 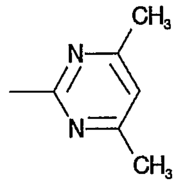 |
| $-CH_2CH=CH_2$ | H | 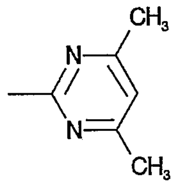 |
| $-CH_3$ | H | 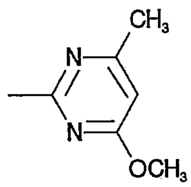 |
| $-C_2H_5$ | H | 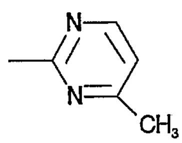 |
| $-CH_2CH=CH_2$ | H | 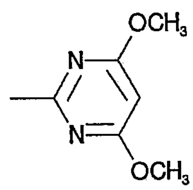 |
| $-CH_2CH(CH_3)_2$ | H | 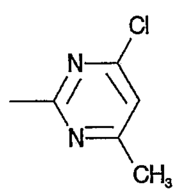 |
| $-CH_3$ | H | 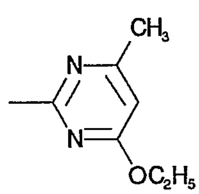 |
| $-C_2H_5$ | H | 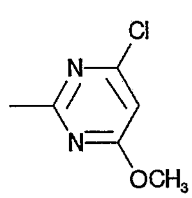 |

Tabelle 1 (Fortsetzung)

| $R^1$ | $R^2$ | $R^3$ |
|---|---|---|
| $-CH_3$ | H | 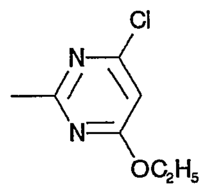 |
| $-CH_2-$ 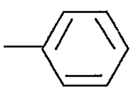 | H | 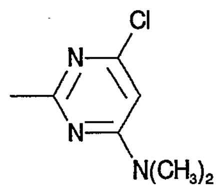 |
| $-CH_3$ | H | 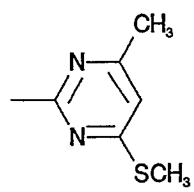 |
| $-C_2H_5$ | H | 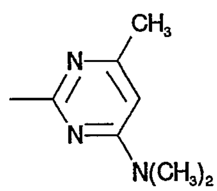 |
| $-CH_3$ | H | 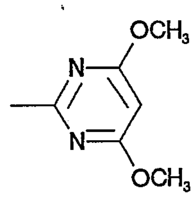 |
| $-CH_2CH{=}CH_2$ | H | 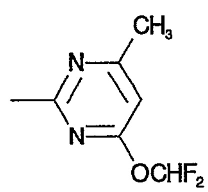 |
| $-CH_3$ | H | 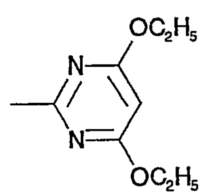 |
| $-CH_3$ | $-C_2H_5$ | 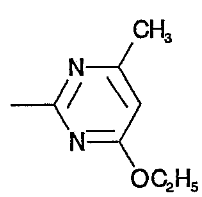 |

Tabelle 1 (Fortsetzung)

| $R^1$ | $R^2$ | $R^3$ |
|---|---|---|
| $-C_3H_7(-n)$ | H | 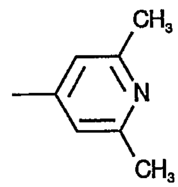 |
| $-CH_2COOC_2H_5$ | H | 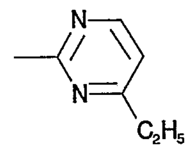 |
| $-CH_3$ | H | 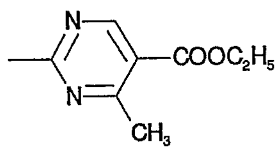 |

Die Verbindungen der Formel (II) sind bisher nicht in der Literatur beschrieben. Man erhält die Verbindungen der Formel (II), wenn man Benzol-1,2-disulfonsäure-dichlorid der Formel (III)

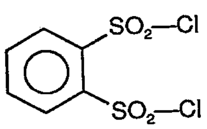

(III)

mit Oxyguanidin-Derivaten der Formel (IV)

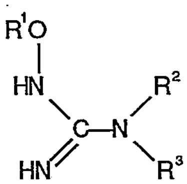

(IV)

in welcher

$R^1$, $R^2$ und $R^3$ die oben angegebene Bedeutung haben, in Gegenwart von Säureakzeptoren, wie z.B. Pyridin oder Diazabicyclooctan (DABCO), und gegebenenfalls in Gegenwart von Verdünnungsmitteln, wie z.B. Methylchlorid, Chloroform, Tetrahydrofuran oder Dioxan, bei Temperaturen zwischen -30°C und +50°C umsetzt.

Die Aufarbeitung kann nach üblichen Methoden erfolgen, beispielsweise durch Einengen, Aufnehmen des Rückstandes in Methylenchlorid, Waschen mit verdünnter Salzsäure und mit Wasser, Abtrennen, Trocknen, Filtrieren und Einengen der organischen Phase, wobei die Produkte der Formel (II) im Rückstand verbleiben.

Das als Ausgangsstoff zu verwendende Benzol-1,2-disulfonsäure-dichlorid der Formel (III) ist bereits bekannt (vgl. J. Org. chem. 31, (1966), 3289–3292).

Die weiter als Ausgangsstoffe zu verwendenden Oxyguanidin-Derivate sind durch die Formel (IV) allgemein definiert. In Formel (IV) haben $R^1$, $R^2$ und $R^3$ vorzugsweise die gleichen Bedeutungen, wie sie oben im Rahmen der Substituentendefinition der Formel (I) vorzugsweise angegeben sind.

Als Ausgangsstoffe der Formel (IV) seien beispielsweise genannt:

N′-(4-Methyl-pyrimidin-2-yl)-, N′-(4-Ethyl-pyrimidin-2-yl)-, N′-(4,6-Dimethoxy-pyrimidin-2-yl)-, N′-(2,6-Dimethyl-pyrimidin-4-yl)-, N′-(4-Difluormethoxy-6-methyl-pyrimidin-2-yl)-, N′-(2,6-Dimethyl-pyrimidin-4-yl)-, N′-(4,6-Dimethyl-pyrimidin-2-yl)-, N′-(4-Methoxy-6-methyl-pyrimidin-2-yl)-, N′-(4-Ethoxy-6-methyl-pyrimidin-2-yl)-, N′-(4-Chlor-6-methoxy-pyrimidin-2-yl)-, N′-(4-Chlor-6-ethoxy-pyrimidin-2-yl)-, N′-(4-Chlor-6-dimethylamino-pyrimidin-2-yl)-, N′-(4-Methyl-6-methylthio-pyrimidin-2-yl)-, und N′-(4-Dimethylamino-6-methyl-pyrimidin-2-yl)-, N″-(methoxy-guanidin, -N″-ethoxy-guanidin, -N″-propoxy-guanidin, -N″-isopropoxyguanidin, -N″-butoxy-guanidin, -N″-isobutoxy-guanidin, -N″-sec.-butoxy-guanidin, -N″-pentoxy-guanidin, -N″-isopentoxy-guanidin, -N″-hexyloxy-guanidin, -N″-octyloxy-guanidin, -N″-allyloxy-guanidin, -N″-(2-chlor-ethoxy)-guanidin, -N″-(2-fluor-ethoxy)-guanidin, -N″-(2-chlor-propoxy)-guanidin, -N″-(2-fluor-propoxy)-guanidin, -N″-(3-chlor-propoxy)-guanidin, -N″-(4-chlor-butoxy)-guanidin, -N″-methoxycarbonylmethoxy-guanidin, -N″-ethoxycarbonyl-methoxy-guanidin, -N″-(1-methoxycarbonyl-ethoxy)-guanidin, -N″-(1-ethoxycarbonylethoxy)-guanidin, -N″-Dimethylaminocarbonylmethoxy-guanidin, -N″-(2-phenyl-ethoxy)-guanidin, -N″-phenoxy-guanidin, -N″-(4-methyl-benzyl-oxy)-guanidin, -N″-(4-fluor-benzyloxy)-guanidin, -N″-(4-chlor-benzyloxy)-guanidin, -N″-(4-nitrobenzyloxy)-guanidin, -N″-(2,6-dichlor-benzyloxy)-guanidin, -N″-(4-methoxy-carbonyl-benzyloxy)-guanidin und -N″-(4-ethoxy-carbonyl-benzyloxy)-guanidin.

Die Ausgangsstoffe der Formel (IV) sind zum Teil bekannt (vgl. J. Chem. Soc. 1962, S. 3915 und DE-OS 3 334 455).

Man erhält die Verbindungen der Formel (IV), wenn man Cyanamid-Derivate der Formel (V)

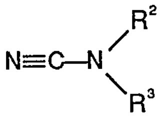 (V)

in welcher
$R^2$ und $R^3$ die oben angegebenen Bedeutungen haben, mit Hydroxylamin-Derivaten der Formel (VI)

$H_2N—OR^1$ (VI)

in welcher
$R^1$ die oben angegebenen Bedeutungen hat, bzw. mit Hydrochloriden von Hydroxylamin-Derivaten der Formel (VI) gegebenenfalls in Gegenwart von Verdünnungsmitteln, wie z.B. Ethanol, Propanol oder Butanol, bei Temperaturen zwischen 20°C und 120°C umsetzt und gegebenenfalls die Umsetzungsprodukte mit Säureakzeptoren, wie z.B. Ammoniak, Kaliumcarbonat oder Natriumhydroxid, behandelt.

Die Cyanamid-Derivate der Formel (V) sind zum Teil bekannt (vgl. J. Chem. Soc. 1953, 1725). Man erhält die Verbindungen der Formel (V) im wesentlichen nach folgenden Synthesewegen:
(a) durch Umsetzung von Alkalimetall- oder Erdalkalimetall-Salzen von Cyanamid – wie z.B. Natriumcyanamid oder Calciumcyanamid – mit Chlor-hetarenen der Formel (VII)

$Cl—R^3$ (VII)

in welcher
$R^3$ die oben angegebene Bedeutung hat, und gegebenenfalls anschliessend – wenn $R^2$ nicht für Wasserstoff steht – mit Halogenverbindungen der Formel (VIII)

$Q—R^2$ (VIII)

in welcher
$R^2$ für einen oben angegebenen steht, und
Q für Chlor, Brom oder Iod steht, gegebenenfalls in Gegenwart von inerten Verdünnungsmitteln, wie z.B. Aceton, Acetonitril oder Dimethylformamid, bei Temperaturen zwischen 0°C und 100°C.

Nach Einengen und Auflösen des Rückstandes in Wasser können die Cyanamid-Derivate der Formel (V) durch Ansäuren, z.B. mit Salzsäure, ausgefällt und durch Absaugen isoliert werden.

Alternativ erhält man die Verbindungen der Formel (V)
(b) für den Fall, dass $R^3$ für einen substituierten Pyrimidinylrest steht, durch Umsetzung von Cyanoguanidin ('Dicyandiamid') mit β-Dicarbonylverbindungen oder deren Derivaten, wie Acetylaceton (vgl. J. Chem. Soc. 1953, 1725–1730); Acetessigsäureestern (vgl. J. Prakt. Chem. 77 (1908), 542 und J. Chem. Soc. 1948, 586) oder Malonsäureestern (vgl. De-PS 158 591).

Die aus Acetessigsäureestern bzw. Malonsäureestern erhaltenen 2-Cyanamino-4-hydroxy-6-methyl- bzw. -4,6-dihydroxy-pyramidine können auf bekannte Weise durch Umsetzung mit Alkylierungsmitteln, wie z.B. Dimethyl- oder Diethylsulfat, gegebenenfalls in Gegenwart von Verdünnungsmitteln, wie z.B. Wasser, Methanol, Ethanol, n- und iso-Propanol, Aceton, Dioxan oder Dimethylformamid, und in Gegewart von Säurebindemitteln, wie z.B. Natrium- oder Kalium-hydroxid, Natrium- oder Kalium-carbonat, in entsprechende 2-Cyan-amino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z.B. Acetanhydrid oder Acetylchlorid acyliert und nach der Alkylierung mit wässrigen Säuren oder Basen wieder entacyliert.

In einem weiteren Alternativverfahren erhält man die Verbindungen der Formel (V), wenn man
(c) Amino-hetarene der Formel (IX)

$H_2N—R^3$ (IX)

in welcher
$R^3$ die oben angegebene Bedeutung hat, mit Carbonylisothiocyanaten der Formel (X)

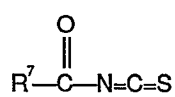 (X)

in welcher
$R^7$ für Ethoxy oder Phenyl steht,
gegebenenfalls in Gegenwart eines inerten Verdünnungsmittels, wie z.B. Aceton, Acetonitril oder Toluol, bei Temperaturen zwischen 0°C und 100°C umsetzt, die hierbei gebildeten Carbonylthioharnstoffe der Formel (XI)

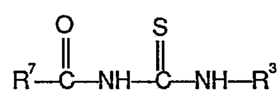 (XI)

in welcher
$R^3$ und $R^7$ die oben angegebenen Bedeutungen haben, gegebenenfalls nach Einengen durch Absaugen isoliert und mit wässrigen Alkalimetall- oder Erdalkalimetallhydroxidlösungen, wie z.B. Natronlauge, gegebenenfalls in Gegenwart eines organischen Lösungsmittels, wie z.B. Tetrahydrofuran oder Dioxan, bei Temperaturen zwischen 0°C und 120°C umsetzt und die nach Ansäuren, z.B. mit Salzsäure, kristallin erhaltenen Thioharnstoffe der Formel (XII)

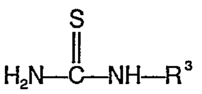

(XII)

in welcher
$R^3$ die oben angegebene Bedeutung hat, durch Absaugen isoliert und mit Metallverbindungen, welche Schwefelwasserstoff binden können, wie z.B. mit Blei(II)-acetat, Kupfer(II)-acetat, Quecksilber(II)-acetat oder Eisen(II)-acetat, in Gegenwart von wässrigen Alkalimetall- oder Erdalkalimetall-hydroxidlösungen, wie z.B. Natronlauge, bei Temperaturen zwischen 20°C und 100°C umsetzt, nach Ende der Umsetzung filtriert und das Filtrat mit einer Säure, wie z.B. Essigsäure, ansäuert. Die hierbei kristallin anfallenden Produkte der Formel (V) können durch Absaugen isoliert werden.

Die Ausgangsstoffe für die vorausgehend unter (a), (b) und (c) beschriebenen Herstellungsverfahren für die Cyanamid-Derivate der Formel (V) sind bekannt und/oder können nach an sich bekannten Verfahren hergestellt werden.

Hierzu gehören die Chlor-hetarene der Formel (VII) [vgl. J. Chem. Soc. (c) 1966, 2031; Chem. Pharm. Bull. 11 (1963), 1382–1388 und Arch. Pharm. 295, (1962), 649–657], die Halogenverbindungen der Formel (VIII) (handelsübliche Chemikalien), die Amino-hetarene der Formel (IX) [vgl. Chem. Pharm. Bull. 11, (1963) S. 1382–1388; J. Chem. Soc. 1946, 81 und US-PS 4 299 960] und die Carbonylisothiocyanate der Formel (X) [vgl. J. Heterocyl. Chem. 5, (1968), 837 und US-PS 4 160 037].

Das beim erfindungsgemässen Verfahren (b) als Ausgangsstoff zu verwendende Benzol-1,2-disulfonsäurechlorid der Formel (III) ist bereits bekannt [vgl. J. Org. Chem. 31, (1966), 3289–3292].

Die beim erfindungsgemässen Verfahren (b) als Ausgangsstoffe zu verwendenden Oxyguanidin-Derivate sind durch die Formel (IV) allgemein definiert. In Formel (IV) haben $R^1$, $R^2$ und $R^3$ vorzugsweise die gleichen Bedeutungen, wie sie oben im Rahmen der Substituentendefinition der Formel (I) vorzugsweise angegeben sind.

Beispiele für Verbindungen der Formel (IV) wurden bereits weiter oben im Zusammenhang mit der Beschreibung der Ausgangsstoffe für Verfahren (a) genannt. Die Herstellung der Ausgangsstoffe der Formel (IV) wurde bereits oben im Zusammenhang mit der Beschreibung der Ausgangsstoffe für Verfahren (a) beschrieben.

Das erfindungsgemässe Verfahren (a) zur Herstellung der neuen Verbindungen der Formel (I) wird vorzugsweise in Wasser als Lösungsmittel durchgeführt. Als weitere Verdünnungsmittel kommen alle inerten organischen Lösungsmittel, vorzugsweise jedoch aprotische polare Solventien in Betracht. Hierzu gehören gegebenenfalls Ketone, wie z.B. Aceton und Methylethylketon, Nitrile, wie z.B. Acetonitril und Propionsäurenitril, Dimethylsulfoxid, Sulfolan, 1,2-Dimethoxyethan und Dioxan.

Verfahren (a) wird in Gegenwart von Basen durchgeführt. Als solche kommen vorzugsweise Alkalimetall- oder Erdalkalimetall-hydroxide, wie z.B. Natrium-, Kalium- und Calcium-hydroxid, tertiäre Amine, wie z.B. Triethylamin, N,N-Dimethylanilin und N,N-Dimethyl-benzylamin, sowie Stickstoffheterocyclen, wie z.B. Pyridin oder Diazabicyclooctan (DABCO) in Betracht.

Die Reaktionstemperaturen können beim erfindungsgemässen Verfahren (a) innerhalb eines grösseren Bereiches variiert werden. Im allgemeinen arbeitet man zwischen 0°C und +100°C, vorzugsweise zwischen 10°C und +80°C. Das erfindungsgemässe Verfahren (a) wird im allgemeinen bei Normaldruck durchgeführt.

Zur Durchführung von Verfahren (a) setzt man je Mol Benzodisultam der Formel (II) im allgemeinen zwischen 1 und 100 Mol, vorzugsweise zwischen 5 und 50 Mol Wasser und gegebenenfalls zwischen 1 und 3 Mol, vorzugsweise zwischen 1 und 2 Mol einer Base ein.

Die Reaktionskomponenten werden gewöhnlich bei Raumtemperatur zusammengegeben und das Reaktionsgemisch wird bis zum Reaktionsende gerührt.

Die Aufarbeitung kann auf übliche Weise durchgeführt werden; beispielsweise indem man – z.B. mit Salzsäure – angesäuert, auf etwa die Hälfte des Volumens einengt und das kristallin anfallende Produkt der Formel (I) durch Absaugen isoliert.

Das erfindungsgemässe Verfahren (b) zur Herstellung der neuen Verbindungen der Formel (I) wird vorzugsweise unter Verwendung von Verdünnungsmitteln durchgeführt. Als solche kommen praktisch alle inerten organischen, vorzugsweise aprotisch polare Solventien in Betracht. Hierzu gehören gegebenenfalls substituierte Kohlenwasserstoffe, wie z.B. Methylenchlorid, Chloroform, 1,2-Dichlorethan, Toluol, Xylol und Chlorbenzol, Nitrile, wie z.B. Acetonitril und Propionitril, Ether, wie z.B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.

Als Säureakzeptoren können bei Verfahren (b) praktisch alle üblicherweise verwendeten Säurebindemittel eingesetzt werden. Hierzu gehören insbesondere Alkalimetall- und Erdalkalimetallhydroxide, Alkalimetall- und Erdalkalimetallhydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicyclononen (DBN), Diazabicycloundecen (DBU), Pyridin, 2-Methyl-5-ethyl-pyridin und 4-Dimethylaminopyridin.

Die Reaktionstemperaturen können bei Verfahren (b) innerhalb eines grösseren Bereiches variiert werden. Im allgemeinen arbeitet man zwischen −80°C und +100°C, vorzugsweise zwischen −30°C und +50°C. Das erfindungsgemässe

Verfahren (b) wird im allgemeinen bei Normaldruck durchgeführt.

Zur Durchführung von Verfahren (b) setzt man je Mol Oxyguanidin-Derivat der Formel (IV) im allgemeinen zwischen 1 und 2 Mol, vorzugsweise zwischen 1,0 und 1,2 Mol Benzol-1,2-disulfonsäure-dichlorid der Formel (III) und anschliessend zwischen 1 und 100 Mol, vorzugsweise zwischen 5 und 50 Mol Wasser, sowie gegebenenfalls zwischen 1 und 3 Mol, vorzugsweise zwischen 1 und 2 Mol einer Base ein.

Die Reaktionskomponenten werden gewöhnlich bei Raumtemperatur oder unter Aussenkühlung zusammengegeben und das Reaktionsgemisch wird bis zum Reaktionsende gerührt.

Die Aufarbeitung kann auf übliche Weise durchgeführt werden; beispielsweise indem man gegebenenfalls einengt und/oder mit einem mit Wasser praktisch nicht mischbaren organischen Lösungsmittel, wie z.B. Methylenchlorid, verdünnt, mit verdünnter Salzsäure und mit Wasser wäscht, trocknet, filtriert und einengt. Das im Rückstand verbleibende Produkt der Formel (I) wird durch Verreiben mit einem geeigneten organischen Lösungsmittel, wie z.B. Ethanol, zur Kristallisation gebracht und durch Absaugen isoliert.

Die erfindungsgemässen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: Z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier - und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweisshydrolysate;

als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Für die Mischungen kommen bekannte Herbizide wie z.B. N-(2-Benzthiazolyl)-N-N′-dimethylharnstoff, 3-(3-Chlor-4-methylphenyl)-1,1-dimethylharnstoff, 3-(4-isopropyl-phenyl)-1,1-dimethylharnstoff, 4-Amino-6-(1,1-dimethyl-ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pyridin-2-oxy)-phenoxy]-propionsäure-(trimethylsilyl)-methyl-esters, das R-Enantiomere des 2-[4-(3,5-Dichlorpyridyl-2-oxy)-phenoxy]-propionsäure-(2-benzyloxy)-ethylester, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-bezonitril, 3,5-Dibrom-4-hydroxy-benzonitril sowie Diphenylether und Phenylpyridazine, wie z.B. Pyridate in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfrass, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemässen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem grösseren Bereich schwanken. Sie hängt im wesentlichen von der Art des gewünschten Effektes ab. Im allgemeinen liegen die Aufwandmengen zwischen 0,001 und 15 kg Wirkstoff pro Hektar Bodenfläche, vorzugsweise zwischen 0,005 und 10 kg pro ha.

Die Herstellung und die Verwendung der erfindungsgemässen Wirkstoffe geht aus den nachfolgenden Beispielen hervor.

Herstellungsbeispiele

Beispiel 1

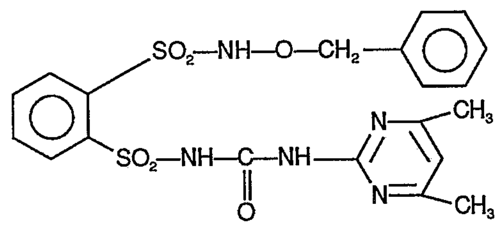

(Verfahren (a))

Eine Mischung von 21,9 g (0,05 Mol) der Verbindung nachstehender Strukturformel

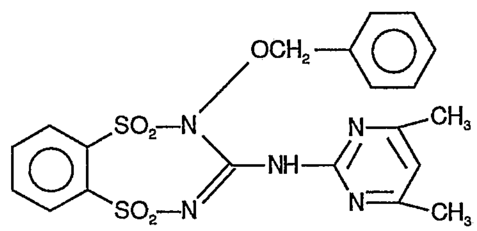

mit 4 g (0,1 Mol) Natriumhydroxid, 50 ml Wasser und 50 ml Dioxan wird 2 Stunden auf 40°C erhitzt.

Dann wird mit konzentrierter Salzsäure angesäuert und die Lösung auf etwa die Hälfte eingeengt. Das kristallin angefallene Produkt wird abgesaugt und getrocknet. Man erhält 12,5 g (54% der Theorie) 1-(2-Benzyloxyaminosulfonylphenylsulfonyl)-3-(4,6-dimethylpyrimidin-2-yl)-harnstoff vom Schmelzpunkt 186°C (Zers.).

Beispiel 2

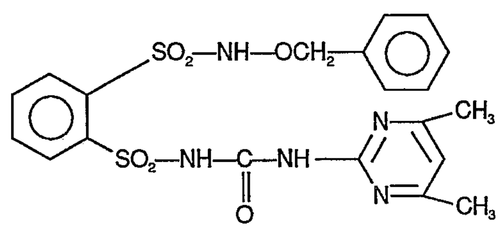

(Verfahren (b))

14 g (0,05 Mol) Benzol-1,2-disulfonsäure-dichlorid werden portionsweise bei −20°C zu einer Mischung von 13,6 g (0.05 Mol) N′-(4,6-Dimethylpyrimidin-2-yl)-N″-benzyl-oxyguanidin, 12 g (0,15 Mol) Pyridin und 200 ml Methylenchlorid gegeben. Man rührt 3 Stunden bei −20°C und 15 Stunden bei +20°C nach.

Dann wird das Reaktionsgemisch mit 10 ml Wasser versetzt, und wird noch 2 Stunden bei 20°C gerührt.

Dann wird die Methylenchloridlösung mit verdünnter Salzsäure und Wasser gewaschen und eingeengt. Der Rückstand wird mit Ethanol verrieben; das kristallin angefallene Produkt wird durch Absaugen isoliert.

Man erhält 6,2 g (27% der Theorie) 1-(2-Benzyloxyaminosulfonylphenylsulfonyl)-3-(4,6-dimethylpyrimidin-2-yl)-harnstoff vom Schmelzpunkt 188°C (Zers.).

Nach dem in den vorausgehenden Beispielen 1 und 2 exemplarisch beschriebenen Verfahren können auch die in der nachstehenden Tabelle 2 aufgeführten Verbindungen der Formel (I) hergestellt werden:

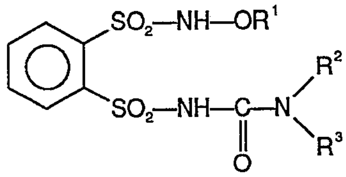

(I)

Tabelle 2

| Bsp. Nr. | $R^1$ | $R^2$ | $R^3$ | Schmelzpunkt (°C) |
|---|---|---|---|---|
| 3 | $-CH_2-CH=CH_2$ | H | 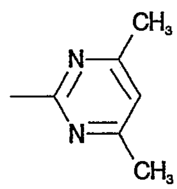 | 182 (Zers.) |
| 4 | $-C_8H_{17}(-n)$ | H | 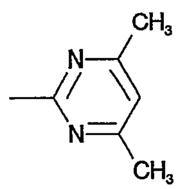 | 145 |
| 5 | 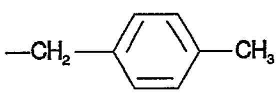 | H | 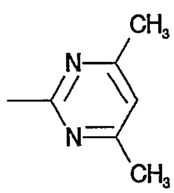 | |
| 6 | 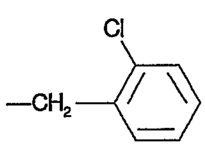 | H | 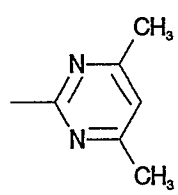 | |
| 7 | 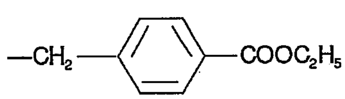 | H | 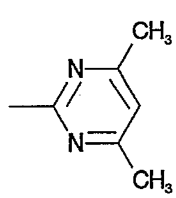 | |
| 8 | 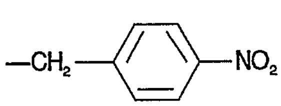 | H | 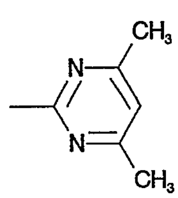 | |

Tabelle 2 (Fortsetzung)

| Bsp. Nr. | $R^1$ | $R^2$ | $R^3$ | Schmelzpunkt (°C) |
|---|---|---|---|---|
| 9 | $—CH_2CH_2—$ 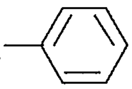 | H | 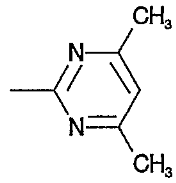 | |
| 10 | $—CH_2—COOC_2H_5$ | H | 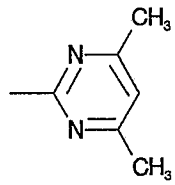 | |
| 11 | $—CH(CH_3)—COOCH_3$ | H | 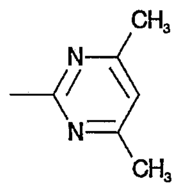 | |
| 12 | $—CH_2CH(CH_3)_2$ | H | 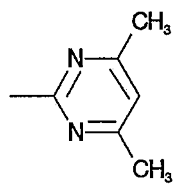 | |
| 13 | $—CH_3$ | $CH_3$ | 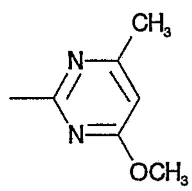 | |
| 14 | $—CH_3$ | H | 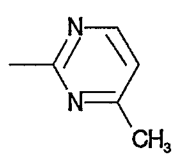 | 194–195 |
| 15 | $—C_2H_5$ | H | 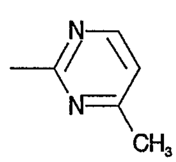 | |
| 16 | $—CH_2—CH=CH_2$ | H | 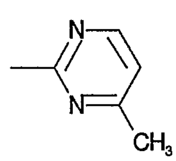 | |

Tabelle 2 (Fortsetzung)

| Bsp. Nr. | $R^1$ | $R^2$ | $R^3$ | Schmelzpunkt (°C) |
|---|---|---|---|---|
| 17 | $-CH_2-$ 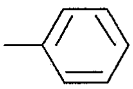 | H | 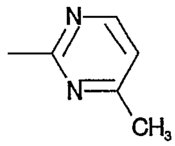 | |
| 18 | $-CH_2COOC_2H_5$ | H | 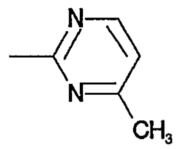 | |
| 19 | $-CH_2CH(CH_3)_2$ | H | 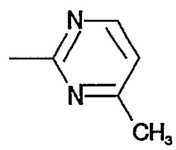 | |
| 20 | $-CH_3$ | H | 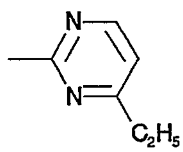 | |
| 21 | $-CH_3$ | H | 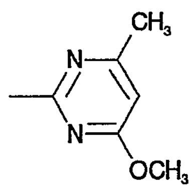 | 218 (Zers.) |
| 22 | $-C_2H_5$ | H | 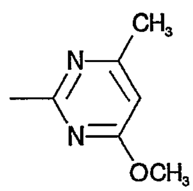 | |
| 23 | $-C_3H_7$ | H | 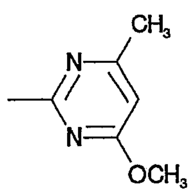 | |
| 24 | $-CH_2-CH{=}CH_2$ | H | 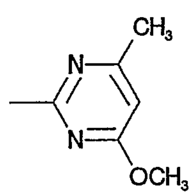 | |

Tabelle 2 (Fortsetzung)

| Bsp. Nr. | $R^1$ | $R^2$ | $R^3$ | Schmelzpunkt (°C) |
|---|---|---|---|---|
| 25 | $—CH_2CH(CH_3)_2$ | H | 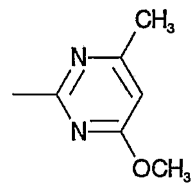 | |
| 26 | $—CH_2—$ 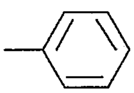 | H | 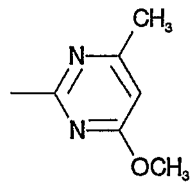 | |
| 27 | $—CH_3$ | H | 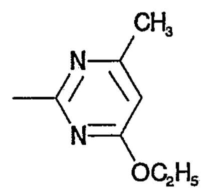 | |
| 28 | $—CH_3$ | H | 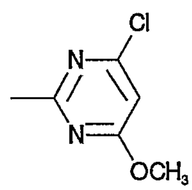 | |
| 29 | $—C_2H_5$ | H | 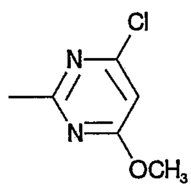 | |
| 30 | $—CH_3$ | H | 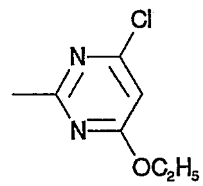 | |
| 31 | $—CH_3$ | H | 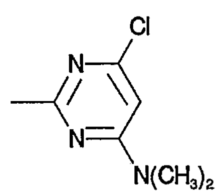 | |
| 32 | $—CH_3$ | H | 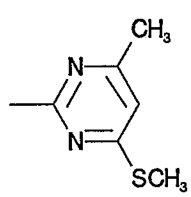 | |

Tabelle 2 (Fortsetzung)

| Bsp. Nr. | $R^1$ | $R^2$ | $R^3$ | Schmelzpunkt (°C) |
|---|---|---|---|---|
| 33 | $-CH_3$ | H | 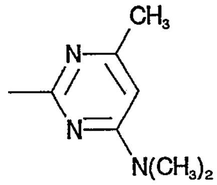 | |
| 34 | $-CH_3$ | H | 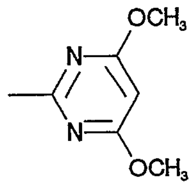 | |
| 35 | $-C_2H_5$ | H | 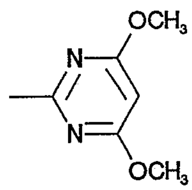 | |
| 36 | $-C_3H_7$ | H | 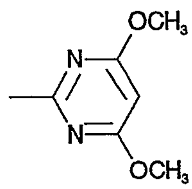 | |
| 37 | $-CH_2-CH=CH_2$ | H | 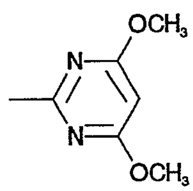 | |
| 38 | $-CH_2CH(CH_3)_2$ | H | 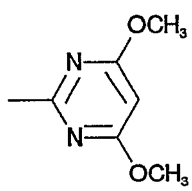 | |
| 39 | $-CH_2-$ 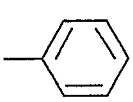 | H | 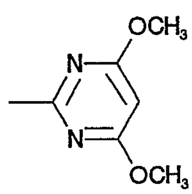 | |
| 40 | $-C_4H_9$ | H | 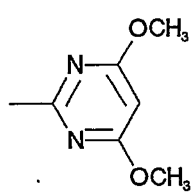 | |

Tabelle 2 (Fortsetzung)

| Bsp. Nr. | $R^1$ | $R^2$ | $R^3$ | Schmelzpunkt (°C) |
|---|---|---|---|---|
| 41 | $-CH_3$ | H | 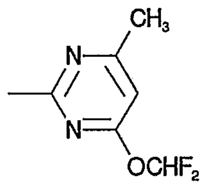 | |
| 42 | $-C_2H_5$ | H | 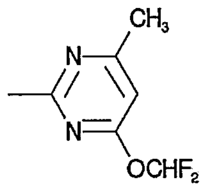 | |
| 43 | $-CH_3$ | H | 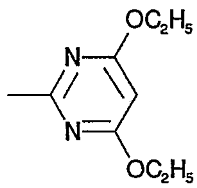 | |
| 44 | $-CH_3$ | H | 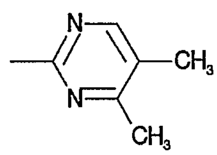 | |
| 45 | $-CH_3$ | H | 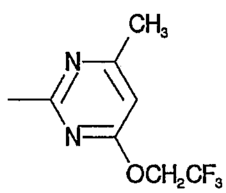 | |
| 46 | $-CH_2-$ 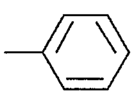 | H | 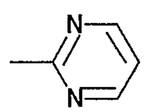 | |
| 47 | $-CH_3$ | H | 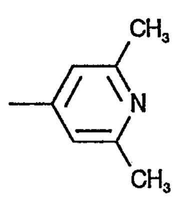 | |
| 48 | $-CH_3$ | H | 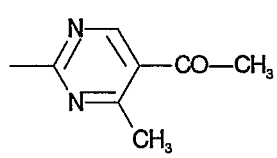 | |

Tabelle 2 (Fortsetzung)

| Bsp. Nr. | $R^1$ | $R^2$ | $R^3$ | Schmelzpunkt (°C) |
|---|---|---|---|---|
| 49 | $-C_2H_5$ | H | 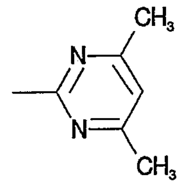 | 218 |
| 50 | $-C_3H_7(-n)$ | H | 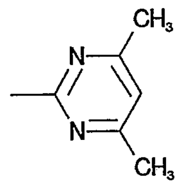 | 212 (Zers.) |
| 51 | $-C_3H_7(-i)$ | H | 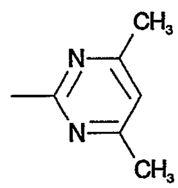 | 218 (Zers.) |
| 52 | $-C_4H_9(-n)$ | H | 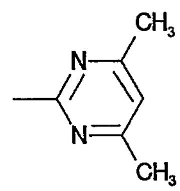 | 109 |
| 53 | $-CH_3$ | H | 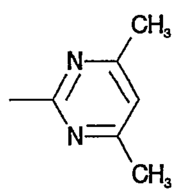 | 218 |

Herstellung der Ausgangsverbindungen der Formel (II)

Beispiel (II-1)

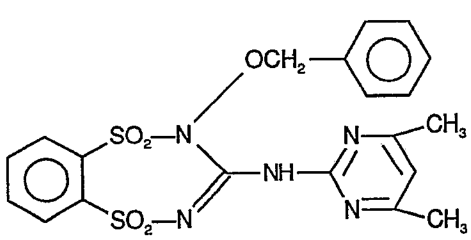

14 g (0,05 Mol) Benzol-1,2-disulfonsäuredichlorid werden portionsweise bei -20°C zu einer Mischung von 13,6 g (0,05 Mol) N′-(4,6-Dimethylpyrimidin-2-yl)-N″-benzyloxy-guanidin, 12 g (0,15 Mol) Pyridin und 100 ml Methylenchlorid gegeben. Man rührt 3 Stunden bei -20°C und 15 Stunden bei +20°C nach.

Dann wird das Reaktionsgemisch eingedampft und der Rückstand mit 70 ml Dioxan versetzt. Es wird filtriert. Das Filtrat wird eingeengt, mit Ethanol verrieben und das ausgefallene Produkt durch Absaugen isoliert.

Man erhält 15 g (68% der Theorie) der Verbindung der oben angegebenen Strukturformel vom Schmelzpunkt 199°C.

Nach dem im vorausgehenden Beispiel exemplarisch beschriebenen Verfahren können die in der nachstehenden Tabelle 3 aufgeführten Verbindungen der Formel (II) hergestellt werden:

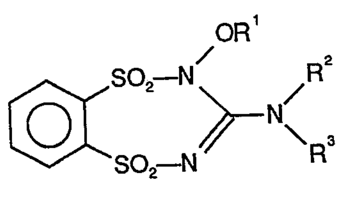

(II)

Tabelle 3

| Bsp. Nr. | $R^1$ | $R^2$ | $R^3$ | Schmelzpunkt (°C) |
|---|---|---|---|---|
| (II–2) | $-CH_2-CH{=}CH_2$ | H | 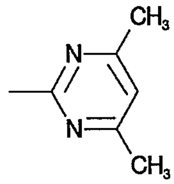 | 180 (Zers.) |
| (II–3) | $-C_8H_{17}(-n)$ | H | 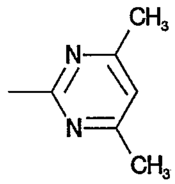 | 164 |
| (II–4) | $-CH_2-COOC_2H_5$ | H | 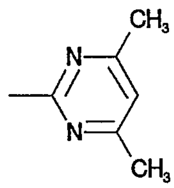 | 210 (Zers.) |
| (II–5) | 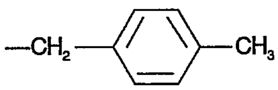 | H | 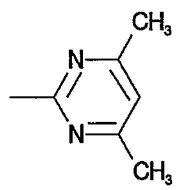 | |
| (II–6) | 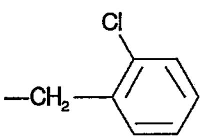 | H | 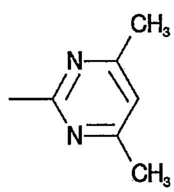 | |
| (II–7) | 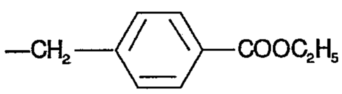 | H | 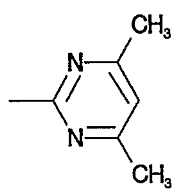 | |
| (II–8) | 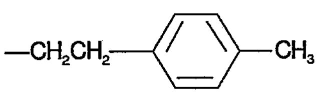 | H | 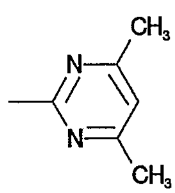 | |
| (II–9) | 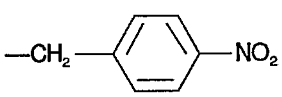 | H | 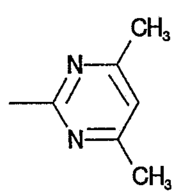 | |

Tabelle 3 (Fortsetzung)

| Bsp. Nr. | $R^1$ | $R^2$ | $R^3$ | Schmelzpunkt (°C) |
|---|---|---|---|---|
| (II–10) | 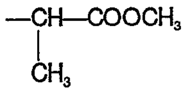 | H | 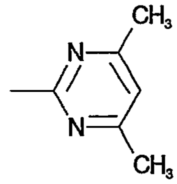 | |
| (II–11) | $-CH_2-CH(CH_3)_2$ | H | 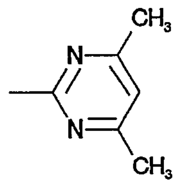 | amorph |
| (II–12) | $-CH_3$ | $CH_3$ | 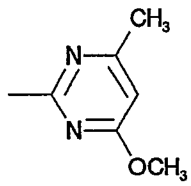 | |
| (II–13) | $-CH_3$ | $C_2H_5$ | 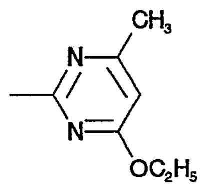 | |
| (II–14) | $-CH_3$ | H | 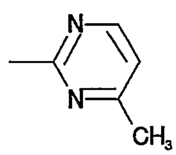 | |
| (II–15) | $-CH_2CH(CH_3)_2$ | H | 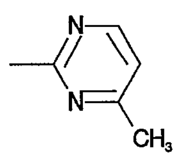 | |
| (II–16) | 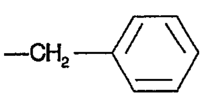 | H | 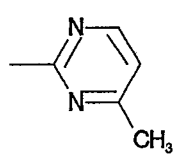 | |
| (II–17) | $-CH_2-COOC_2H_5$ | H | 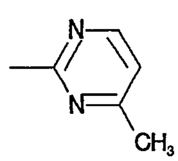 | |

Tabelle 3 (Fortsetzung)

| Bsp. Nr. | $R^1$ | $R^2$ | $R^3$ | Schmelzpunkt (°C) |
|---|---|---|---|---|
| (II–18) | $—CH_3$ | H | 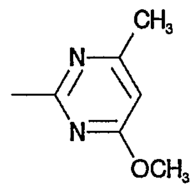 | 151 (Zers.) |
| (II–19) | $—C_2H_5$ | H | 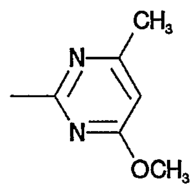 | |
| (II–20) | $—C_3H_7$ | H | 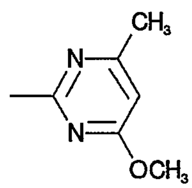 | |
| (II–21) | $—CH_2—CH{=}CH_2$ | H | 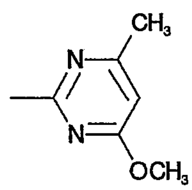 | |
| (II–22) | $—CH_2CH(CH_3)_2$ | H | 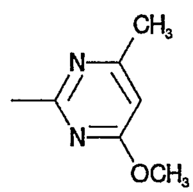 | |
| (II–23) | $—CH_2—$ 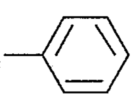 | H | 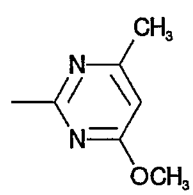 | |
| (II–24) | $—CH_3$ | H | 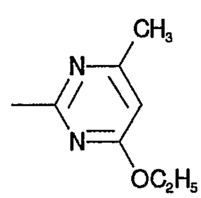 | |
| (II–25) | $—CH_3$ | H | 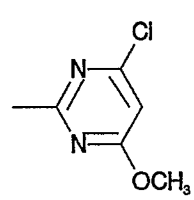 | |

Tabelle 3 (Fortsetzung)

| Bsp. Nr. | $R^1$ | $R^2$ | $R^3$ | Schmelzpunkt (°C) |
|---|---|---|---|---|
| (II–26) | $-CH_3$ | H | 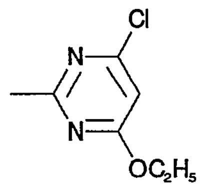 | |
| (II–27) | $-CH_3$ | H | 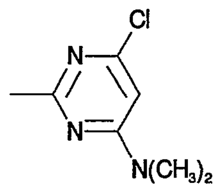 | |
| (II–28) | $-CH_3$ | H | 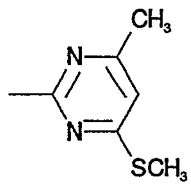 | |
| (II–29) | $-CH_3$ | H | 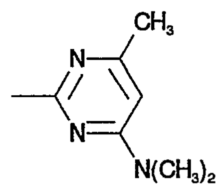 | |
| (II–30) | $-CH_3$ | H | 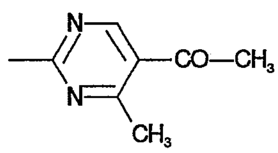 | |
| (II–31) | $-CH_3$ | H | 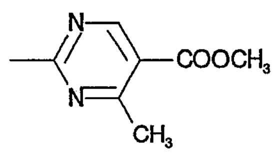 | |
| (II–32) | $-CH_3$ | H | 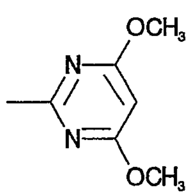 | |
| (II–33) | $-C_2H_5$ | H | 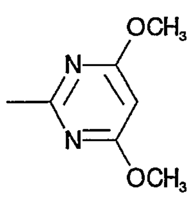 | |

Tabelle 3 (Fortsetzung)

| Bsp. Nr. | $R^1$ | $R^2$ | $R^3$ | Schmelzpunkt (°C) |
|---|---|---|---|---|
| (II–34) | $—C_3H_7(–n)$ | H | 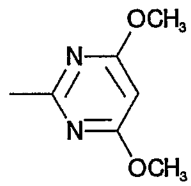 | |
| (II–35) | $—C_4H_9(–n)$ | H | 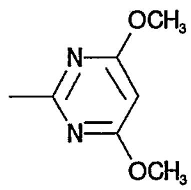 | |
| (II–36) | $—CH_2$ 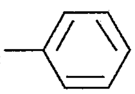 | H | 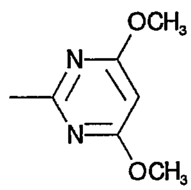 | |
| (II–37) | $—CH_2—CH=CH_2$ | H | 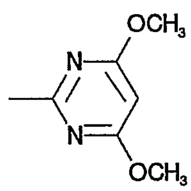 | |
| (II–38) | $—CH_2CH(CH_3)_2$ | H | 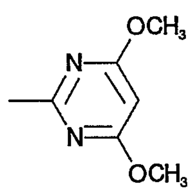 | |
| (II–39) | $—CH_3$ | H | 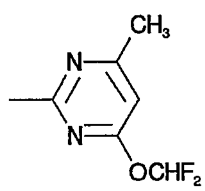 | |
| (II–40) | $—CH_2$ 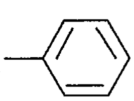 | H | 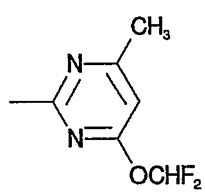 | |
| (II–41) | $—CH_3$ | H | 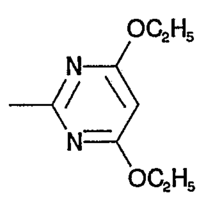 | |

Tabelle 3 (Fortsetzung)

| Bsp. Nr. | $R^1$ | $R^2$ | $R^3$ | Schmelzpunkt (°C) |
|---|---|---|---|---|
| (II–42) | $-CH_3$ | H | 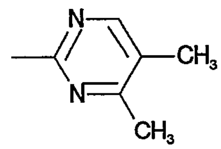 | |
| (II–43) | $-CH_3$ | H | 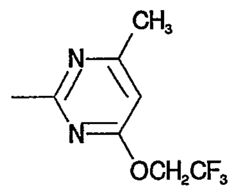 | |
| (II–44) | $-CH_2CH(CH_3)_2$ | H | 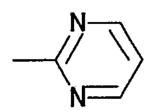 | |
| (II–45) | $-CH_3$ | H | 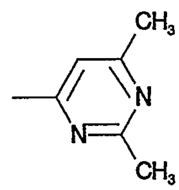 | |
| (II–46) | $-CH_3$ | H | 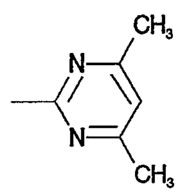 | 158 |
| (II–47) | $-C_2H_5$ | H | 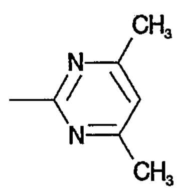 | 104 |
| (II–48) | $-C_3H_7(-n)$ | H | 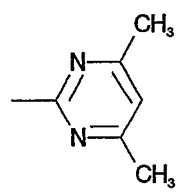 | 134 |
| (II–49) | $-C_4H_9(-n)$ | H | 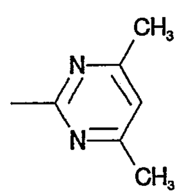 | 179 |

Tabelle 3 (Fortsetzung)

| Bsp. Nr. | $R^1$ | $R^2$ | $R^3$ | Schmelzpunkt (°C) |
|---|---|---|---|---|
| (II–50) | $-CH_3$ | H | 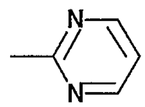 | 187 |

Herstellung der Ausgangsstoffe der Formel (IV)
Beispiel (IV–1)

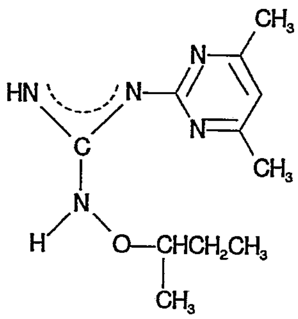

Eine Mischung aus 143 g (0,97 Mol) 2-Cyanamino-4,6-dimethyl-pyrimidin, 94,3 g (1,06 Mol) 0-sec.-Butylhydroxylamin und 190 ml Ethanol wird 6 Stunden unter Rückfluss zum Sieden erhitzt. Dann wird abgesaugt, das Filtrat eingeengt und der Rückstand mit 500 ml Wasser versetzt. Das hierbei kristallin angefallene Produkt wird durch Absaugen isoliert.

Man erhält 131 g (57% der Theorie) N′-(4,6-Dimethyl-pyrimidin-2-yl)-N″-sec.-butoxy-guanidin vom Schmelzpunkt 78°C.

Analog können die in der nachstehenden Tabelle 4 aufgeführten Verbindungen der Formel (IV) hergestellt werden:

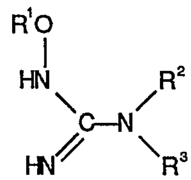

(IV)

Tabelle 4

| Bsp. Nr. | $R^1$ | $R^2$ | $R^3$ | Schmelzpunkt (°C) |
|---|---|---|---|---|
| (IV–2) | 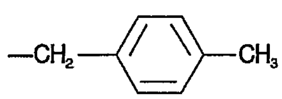 | H | 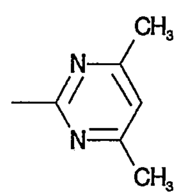 | 85–86 |
| (VI–3) | 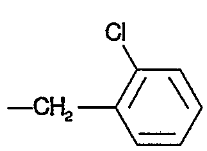 | H | 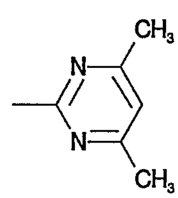 | 102–103 |
| (IV–4) | 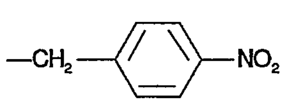 | H | 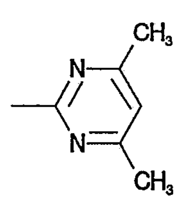 | 170–172 |

Tabelle 4 (Fortsetzung)

| Bsp. Nr. | $R^1$ | $R^2$ | $R^3$ | Schmelzpunkt (°C) |
|---|---|---|---|---|
| (IV–5) | $—CH_2—CH_2—$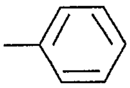 | H | 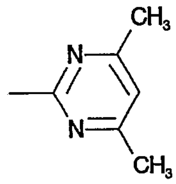 | $n_D^{24.5} = 1.5776$ |
| (IV–6) | $—CH_2—CH{=}CH_2$ | H | 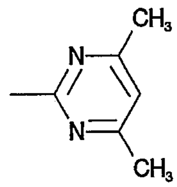 | 103 |
| (IV–7) | $—C_8H_{17}(–n)$ | H | 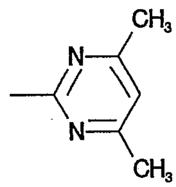 | 58 |
| (IV–8) | $—CH_2—COOC_2H_5$ | H | 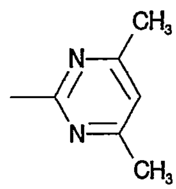 | 98–99 |
| (IV–9) | 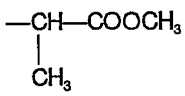 | H | 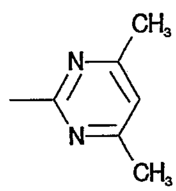 | 147–148 |
| (IV–10) | $—CH_3$ | $—CH_3$ | 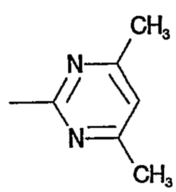 | 95 |
| (VI–11) | $—CH_2CH(CH_3)_2$ | H | 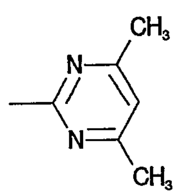 | 52 |
| (IV–12) | 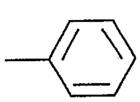 | H | 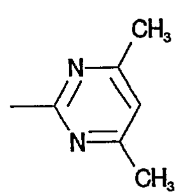 | 189–192 (Zers.) |

Tabelle 4 (Fortsetzung)

| Bsp. Nr. | R¹ | R² | R³ | Schmelzpunkt (°C) |
|---|---|---|---|---|
| (IV–13) | $-CH_2CH_2CH_2Cl$ | H | 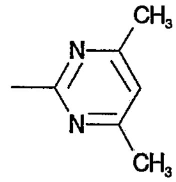 | 137 |
| (IV–14) | $-CH_2COOCH_3$ | H | 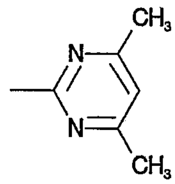 | 148–149 |
| (IV–15) | $-CH_2-$ 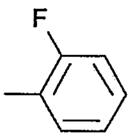 | H | 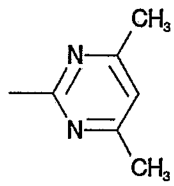 | 114–116 |
| (IV–16) | 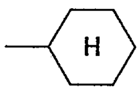 | H | 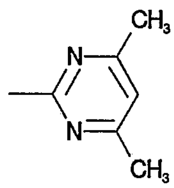 | |
| (IV–17) | $-CH_2-$ 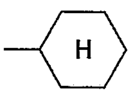 | H | 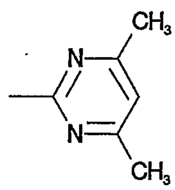 | |
| (IV–18) | $-CH_2-CO-N(CH_3)_2$ | H | 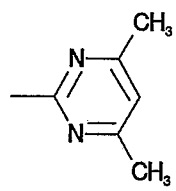 | |
| (VI–19) | $-CH_2OCH_3$ | H | 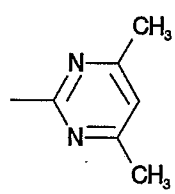 | |
| (IV–20) | $-CH_2SCH_3$ | H | 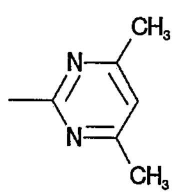 | |

Tabelle 4 (Fortsetzung)

| Bsp. Nr. | $R^1$ | $R^2$ | $R^3$ | Schmelzpunkt (°C) |
|---|---|---|---|---|
| (IV–21) | 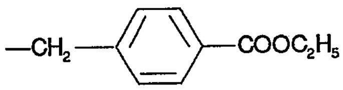 | H | 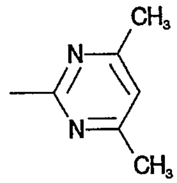 | 138 |
| (IV–22) | $-CH_2CH_2OCH_3$ | H | 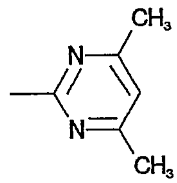 | |
| (IV–23) | $-CH_2CF_3$ | H | 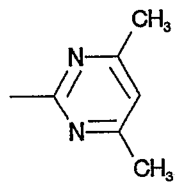 | |
| (IV–24) | 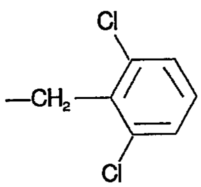 | H | 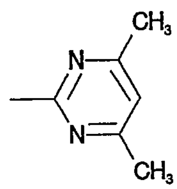 | 152 |
| (IV–25) | 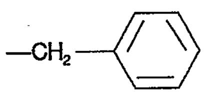 | H | 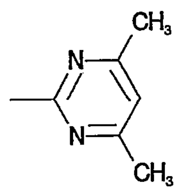 | 102 |
| (IV–26) | $-CH_2-COOCH(CH_3)_2$ | H | 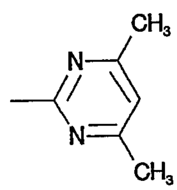 | 112 |
| (VI–27) | $-CH_3$ | H | 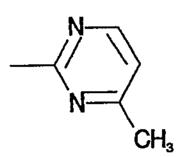 | 152 |
| (IV–28) | $-C_2H_5$ | H | 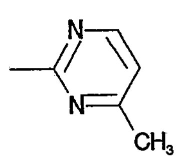 | 95 |

Tabelle 4 (Fortsetzung)

| Bsp. Nr. | $R^1$ | $R^2$ | $R^3$ | Schmelzpunkt (°C) |
|---|---|---|---|---|
| (IV–29) | $—C_3H_7(–n)$ | H | 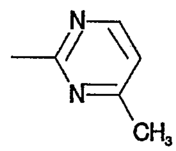 | |
| (IV–30) | $—CH(CH_3)_2$ | H | 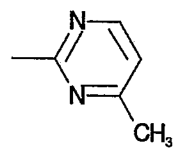 | |
| (IV–31) | $—C_4H_9(–n)$ | H | 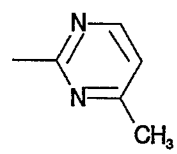 | |
| (IV–32) | $—CH_2CH(CH_3)_2$ | H | 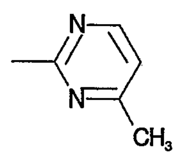 | |
| (IV–33) | $—CH_2—CH=CH_2$ | H | 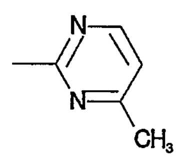 | |
| (IV–34) | $—CH_2—$ 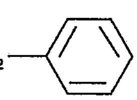 | H | 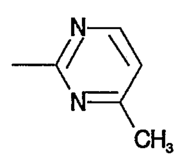 | 150 |
| (VI–35) | $—CH_2—COOC_2H_5$ | H | 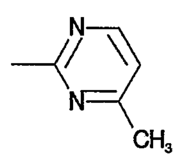 | |
| (IV–36) | $—CH_3$ | H | 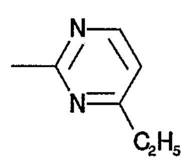 | 98 |

Tabelle 4 (Fortsetzung)

| Bsp. Nr. | $R^1$ | $R^2$ | $R^3$ | Schmelzpunkt (°C) |
|---|---|---|---|---|
| (IV–37) | $—CH_3$ | H | 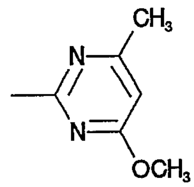 | 126 |
| (IV–38) | $—CH_2—$ 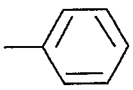 | H | 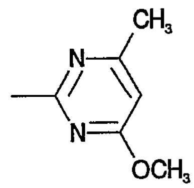 | (amorph) |
| (IV–39) | $—C_2H_5$ | H | 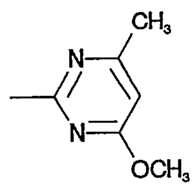 | (amorph) |
| (IV–40) | $—C_3H_7$ | H | 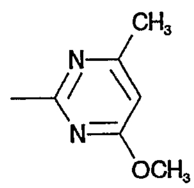 | |
| (IV–41) | $—CH_2CH(CH_3)_2$ | H | 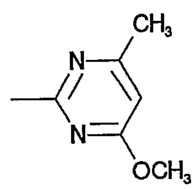 | |
| (IV–42) | $—CH_2—CH{=}CH_2$ | H | 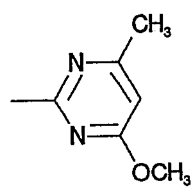 | |
| (VI–43) | $—CH_3$ | H | 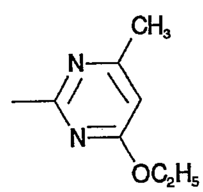 | |
| (IV–44) | $—CH_3$ | $CH_3$ | 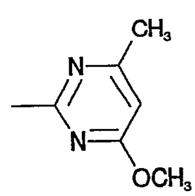 | 135 |

Tabelle 4 (Fortsetzung)

| Bsp. Nr. | $R^1$ | $R^2$ | $R^3$ | Schmelzpunkt (°C) |
|---|---|---|---|---|
| (IV–45) | $-CH_3$ | $C_2H_5$ | 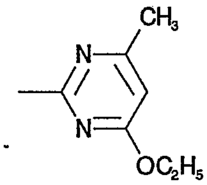 | |
| (IV–46) | $-CH_3$ | H | 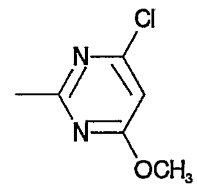 | 112 |
| (IV–47) | $-CH_2-$ 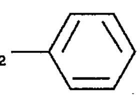 | H | 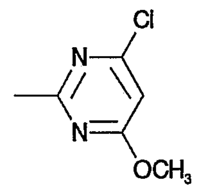 | |
| (IV–48) | $-CH_3$ | H | 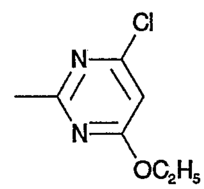 | |
| (IV–49) | $-CH_3$ | H | 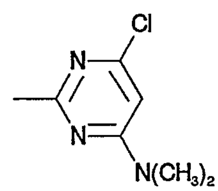 | |
| (IV–50) | $-CH_3$ | H | 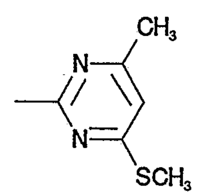 | |
| (VI–51) | $-CH_3$ | H | 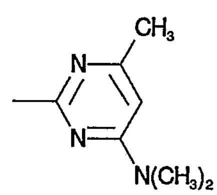 | |
| (IV–52) | $-CH_3$ | H | 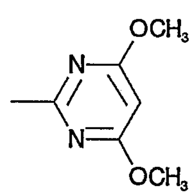 | 122 |

Tabelle 4 (Fortsetzung)

| Bsp. Nr. | $R^1$ | $R^2$ | $R^3$ | Schmelzpunkt (°C) |
|---|---|---|---|---|
| (IV–53) | $—C_2H_5$ | H | 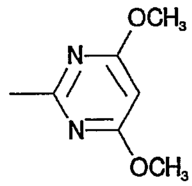 | |
| (IV–54) | $—C_3H_7$ | H | 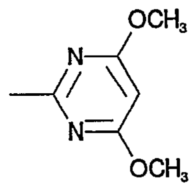 | |
| (IV–55) | $—C_4H_9$ | H | 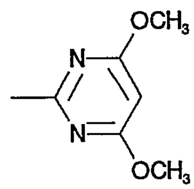 | |
| (IV–56) | $—CH_2CH(CH_3)_2$ | H | 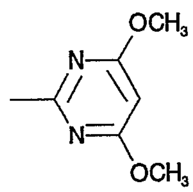 | 76 |
| (IV–57) | $—CH—CH_2CH_3$ (mit $CH_3$ am CH) | H | 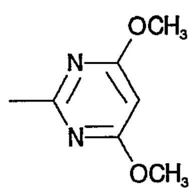 | 68 |
| (IV–58) | $—CH_2$ 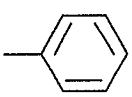 | H | 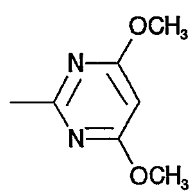 | 74 |
| (VI–59) | $—CH_2—COOC_2H_5$ | H | 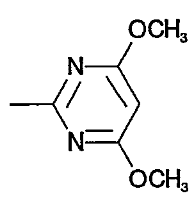 | |
| (IV–60) | $—CH_3$ | H | 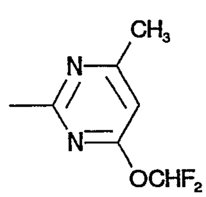 | |

Tabelle 4 (Fortsetzung)

| Bsp. Nr. | $R^1$ | $R^2$ | $R^3$ | Schmelzpunkt (°C) |
| --- | --- | --- | --- | --- |
| (IV–61) | $—CH_3$ | H | 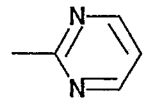 | 107–109 |
| (IV–62) | $—CH_3$ | H | 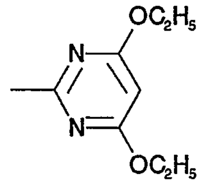 | |
| (IV–63) | 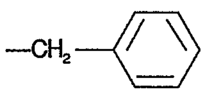 | H | 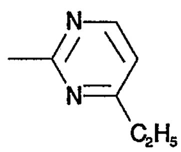 | 112 |
| (IV–64) | $—CH(C_6H_5)_2$ | H | 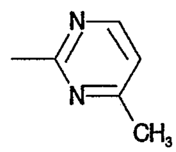 | 165 |
| (IV–65) | 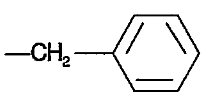 | H | 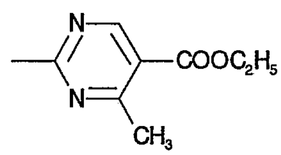 | 130 |

Herstellung der Ausgangsstoffe der Formel (V)

Beispiel (V-1)

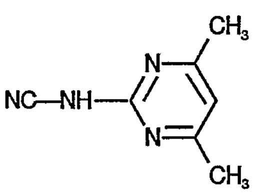

Ein Gemisch aus 42 g (0,5 Mol) Cyanoguanidin ('Dicyandiamid') und 50 g (0,5 Mol) 2,4-Pentandion ('Acetylaceton') wird 15 Stunden auf 120°C erhitzt. Dann wird nach Abkühlen das Reaktionsgemisch mit 500 ml Wasser versetzt und die Lösung bei 0°C bis 10°C mit Salzsäure angesäuert. Das hierbei kristallin angefallene Produkt wird durch Absaugen isoliert. Man erhält 51,8 g (70% der Theorie) 2-Cyanamino-4,6-dimethyl-pyrimidin vom Schmelzpunkt 205°C.

Beispiel (V-2)

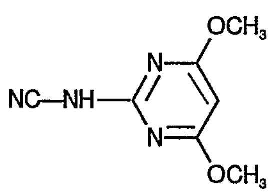

Eine auf 100°C erhitzte Lösung von 24 g (0,427 Mol) Kaliumhydroxid in 100 ml Wasser wird bei 100°C unter Rühren zu einer Mischung von 9,2 g (0,043 Mol) 4,6-Dimethoxy-pyrimidin-2-yl-thioharnstoff und 70 ml Wasser gegeben. Man rührt 2 Minuten bei 100°C nach und gibt dann eine auf 100°C erwärmte Lösung von 16,2 g (0,05 Mol) Blei-II-acetat in 30 ml Wasser hinzu. Man erhitzt noch 5 Minuten unter Rückfluss, kühlt dann auf 0°C bis 5°C ab und versetzt die wässrige Lösung mit 30 ml Eisessig. Das hierbei kristallin ausfallende Produkt wird durch Absaugen isoliert.

Man erhält 6,3 (81,5% der Theorie) 2-Cyanamino-4,6-dimethoxy-pyrimidin vom Schmelzpunkt 202°C.

Nach dem im vorausgehenden Beispiel exemplarisch beschriebenen Verfahren können die in der nachstehenden Tabelle 5 aufgeführten Verbindungen der Formel (V) hergestellt werden:

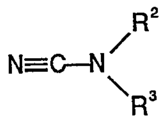 (V)

Tabelle 5

| Bsp. Nr. | $R^2$ | $R^3$ | Schmelzpunkt (°C) |
|---|---|---|---|
| (V–3) | H | 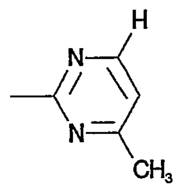 | 203 (Zers.) |
| (V–4) | H | 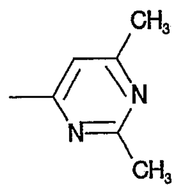 | 234 |
| (V–5) | H | 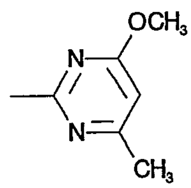 | 258 |
| (V–6) | H | 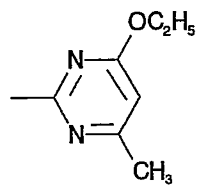 | |
| ( V.-7) | H | 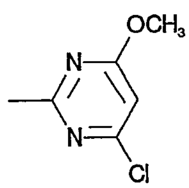 | 200 |
| (V–8) | H | 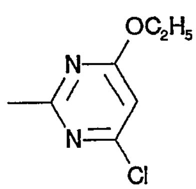 | |
| (V–9) | H | 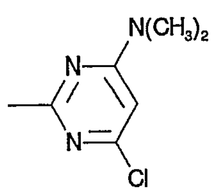 | |

Tabelle 5 (Fortsetzung)

| Bsp. Nr. | $R^2$ | $R^3$ | Schmelzpunkt (°C) |
|---|---|---|---|
| (V–10) | H | 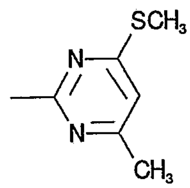 | |
| (V–11) | H | 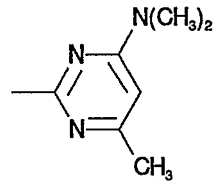 | |
| (V–12) | H | 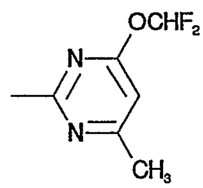 | 174 |
| (V–13) | H | 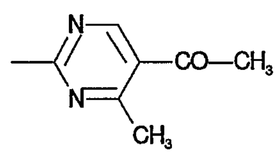 | 174 |
| (V–14) | H | 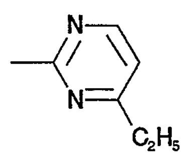 | 146 |
| (V-15) | H | 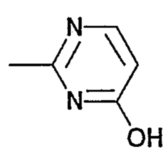 | >300 |
| (V–16) | H | 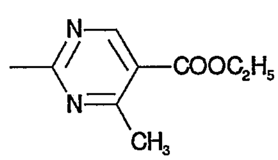 | 126 |
| (V–17) | H | 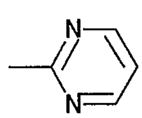 | 186 |

2-(Alkyl-cyano-amino)-pyramidine der Formel (V) können beispielsweise wie folgt hergestellt werden:

Beispiel (V-18)

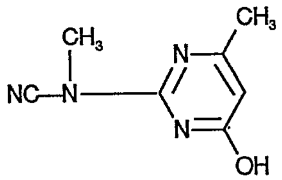

12,6 g (0,1 Mol) Dimethylsulfat werden zu einer Lösung von 15 g (0,1 Mol) 2-Cyanamino-4-hydroxy-6-methyl-pyrimidin und 4,1 g (0,1 Mol) Natriumhydroxid in 60 ml Wasser tropfenweise gegeben, wobei die Reaktionstemperatur von 20°C auf 40°C steigt. Nach zweistündigem Rühren bei 20°C wird das kristallin angefallene Produkt durch Absaugen isoliert.

Man erhält 11,1 g (68% der Theorie) 2-(Methyl-cyanamino)-4-hydroxy-6-methyl-pyrimidin vom Schmelzpunkt 290°C.

Analog erhält man:

Beispiel (V-19)

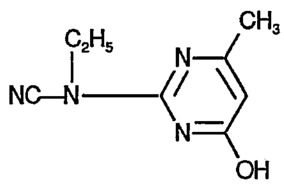

Fp. 215–220°C.

Beispiel (V-20)

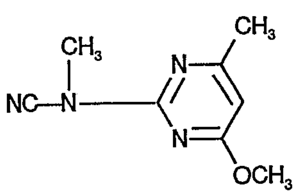

127,4 g (1 Mol) Dimethylsulfat werden zu einer Lösung von 75 g (0,5 Mol) 2-Cyanamino-4-hydroxy-6-methyl-pyrimidin – hergestellt nach Verfahren (b) – und 44 g (1,1 Mol) Natriumhydroxid in 750 ml Wasser tropfenweise gegeben, wobei die Reaktionstemperatur von 20°C auf 35°C ansteigt. Nach zwölfstündigem Rühren bei 20°C wird durch Zugabe von Natronlauge ein pH-Wert zwischen 9 und 10 eingestellt und das kristallin angefallene Produkt durch Absaugen isoliert.

Man erhält 13 g (15% der Theorie) 2-(Methyl-cyano-amino)-4-methoxy-6-methyl-pyrimidin vom Schmelzpunkt 123°C.

Analog erhält man

Beispiel (V-21)

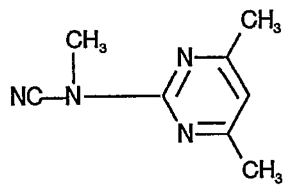

Fp: 104°C.

Beispiel (V-22)

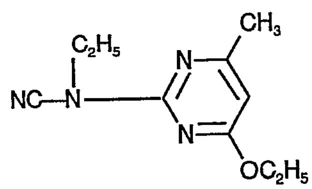

Fp: 71°C.

Herstellung der Ausgangsstoffe der Formel (XI)

Beispiel (XI-1)

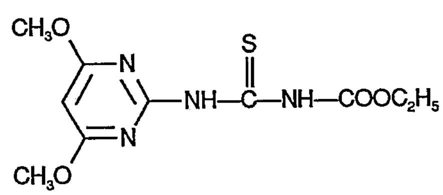

Eine Mischung aus 15,5 g (0,1 Mol) 2-Amino-4,6-dimethoxy-pyrimidin, 13,1 g (0,1 Mol) Ethoxycarbonylisothiocyanat und 200 ml Acetonitril wird 2 Stunden bei 60°C gerührt. Dann wird auf 10°C abgekühlt, und das kristallin angefallene Produkt durch Absaugen isoliert.

Man erhält 22,5 g (79% der Theorie) 1-(Ethoxycarbonyl)-3-(4,6-dimethoxy-pyrimidin-2-yl)-thioharnstoff vom Schmelzpunkt 194°C (Zers.).

Nach dem im vorausgehenden Beispiel exemplarisch beschriebenen Verfahren können die in der nachstehenden Tabelle 6 aufgeführten Verbindungen der Formel (XI) hergestellt werden:

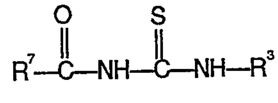

(XI)

Tabelle 6

| Bsp. Nr. | $R^2$ | $R^3$ | Schmelzpunkt (°C) |
|---|---|---|---|
| (XI–2) | 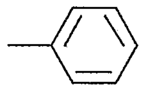 | 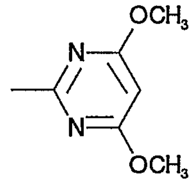 | 189 |
| (XI–3) | 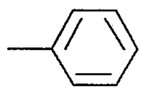 | 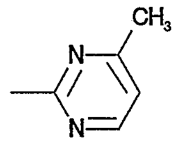 | 198–199 (Zers.) |
| (XI–4) | $—OC_2H_5$ | 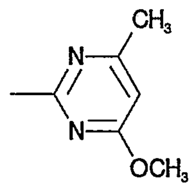 | 217 |
| (XI–5) | 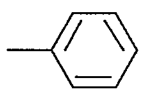 | 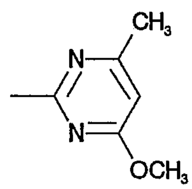 | 190 |
| (XI–6) | $—OC_2H_5$ | 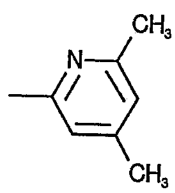 | 140 |
| (XI–7) | 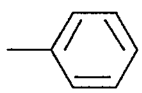 | 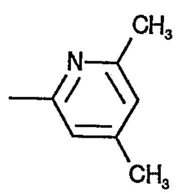 | 145 |
| (XI–8) | 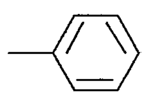 | 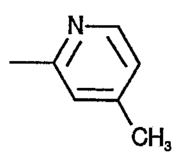 | 161 |
| (XI–9) | $—OC_2H_5$ | 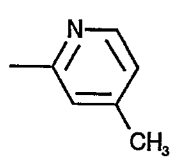 | 119 |

Tabelle 6 (Fortsetzung)

| Bsp. Nr. | $R^2$ | $R^3$ | Schmelzpunkt (°C) |
|---|---|---|---|
| (XI–10) | 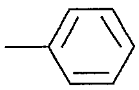 | 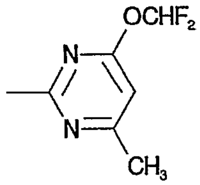 | 182 |
| (XI–11) | $—OC_2H_5$ | 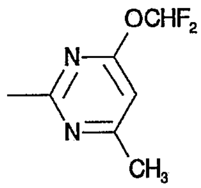 | 184–185 |
| (XI–12) | $—OC_2H_5$ | 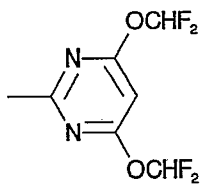 | 173 |
| (XI–13) | $—OC_2H_5$ | 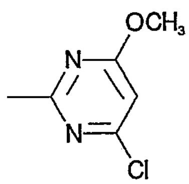 | 160–162 |
| (XI–14) | $—OC_2H_5$ | 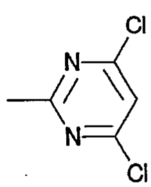 | 132–136 |
| (XI–15) | $—OC_2H_5$ | 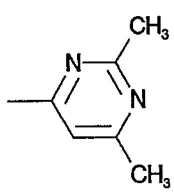 | 169 |
| (XI–16) | 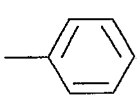 | 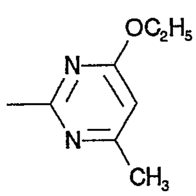 | 156 |
| (XI–17) | $—OC_2H_5$ | 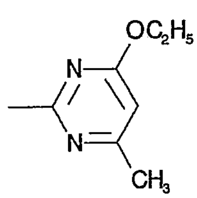 | |

Tabelle 6 (Fortsetzung)

| Bsp. Nr. | $R^2$ | $R^3$ | Schmelzpunkt (°C) |
|---|---|---|---|
| (XI–18) | $-OC_2H_5$ | 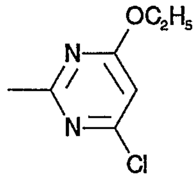 | |
| (XI–19) | 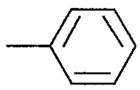 | 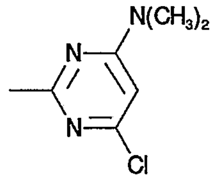 | |
| (XI–20) | $-OC_2H_5$ | 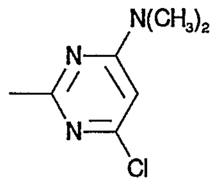 | 168 |
| (XI–21) | $-OC_2H_5$ | 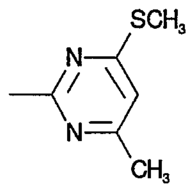 | |
| (XI–22) | 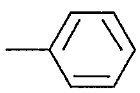 | 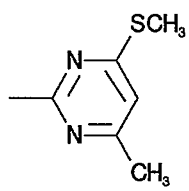 | |
| (XI–23) | $-OC_2H_5$ | 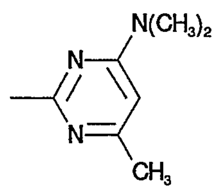 | |
| (XI–24) | 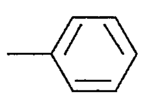 | 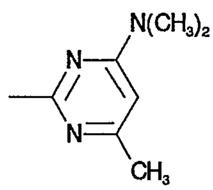 | |
| (XI–25) | $-OC_2H_5$ | 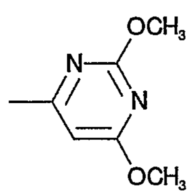 | |

Tabelle 6 (Fortsetzung)

| Bsp. Nr. | $R^2$ | $R^3$ | Schmelzpunkt (°C) |
|---|---|---|---|
| (XI–26) | 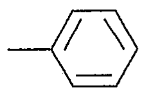 | 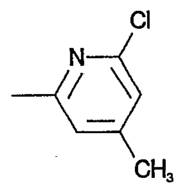 | |
| (XI–27) | $-OC_2H_5$ | 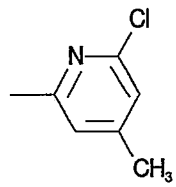 | |
| (XI–28) | 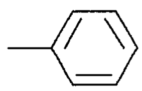 | 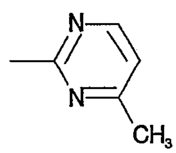 | 173 |
| (XI–29) | 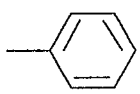 | 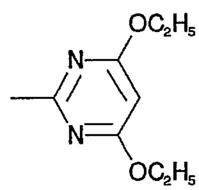 | 179 |

Herstellung der Ausgangsstoffe der Formel (XII)

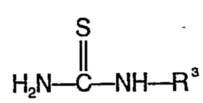 (XII)

Beispiel (XII-1)

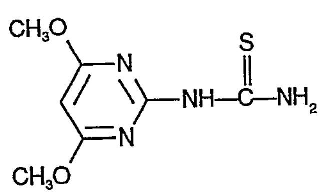

Eine Mischung von 5,0 g (0,0175 Mol) 1-(Ethoxycarbonyl)-3-(4,6-dimethoxy-pyrimidin-2-yl)-thioharnstoff, 4,0 g (0,1 Mol) Natriumhydroxid und 100 ml Wasser wird 2 Tage bei 20°C gerührt. Dann wird unter Rühren solange verdünnte Salzsäure zugetropft, bis die Lösung sauer gestellt ist und die $CO_2$-Entwicklung beendet ist. Das kristallin angefallene Produkt wird durch Absaugen isoliert.

Man erhält 3,5 g (94% der Theorie) 4,6-Dimethoxy-pyrimidin-2-yl-thioharnstoff vom Schmelzpunkt 245–8°C (Zers.).

Nach dem im vorausgehenden Beispiel exemplarisch beschriebenen Verfahren können die in der nachstehenden Tabelle 7 aufgeführten Verbindungen der Formel (XII) hergestellt werden:

Tabelle 7

| Bsp. Nr. | $R^3$ | Schmelzpunkt (°C) |
|---|---|---|
| (XII–2) | 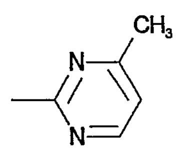 | 264–265 (Zers.) |
| (XII–3) | 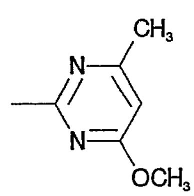 | 231 (Zers.) |
| (XII–4) | 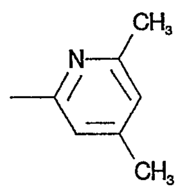 | 259–260 (Zers.) |

Tabelle 7 (Fortsetzung)

| Bsp. Nr. | $R^3$ | Schmelzpunkt (°C) |
|---|---|---|
| (XII–5) | 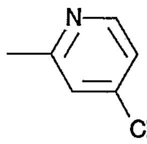 | 214–215 |
| (XII–6) | 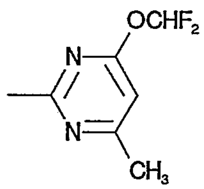 | 192–194 |
| (XII–7) | 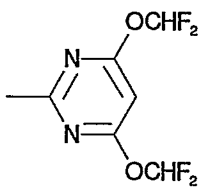 | |
| (XII–8) | 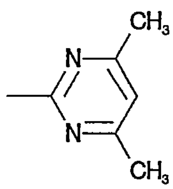 | 225–227 (Zers.) |
| (XII–9) | 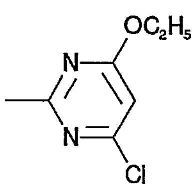 | |
| (XII–10) | 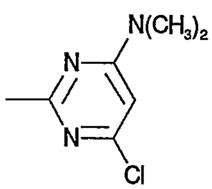 | |
| (XII–11) | 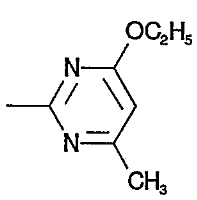 | 156 |
| (XII–12) | 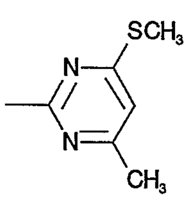 | |

Tabelle 7 (Fortsetzung)

| Bsp. Nr. | $R^3$ | Schmelzpunkt (°C) |
|---|---|---|
| (XII–13) | 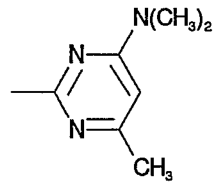 | |
| (XII–14) | 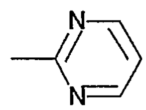 | 263 |
| (XII–15) | 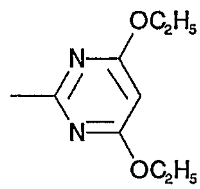 | 166 |
| (XII–16) | 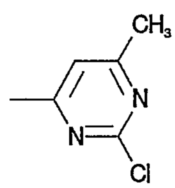 | 248 |

Beispiel A

Pre-emergence-Test

Lösungsmittel: 5 Gewichtsteile Aceton

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Die erfindungsgemässen Wirkstoffe zeigen in diesem Test eine sehr gute herbizide Wirksamkeit.

Beispiel B

Post-emergence-Test

Lösungsmittel: 5 Gewichtsteile Aceton

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, dass die jeweils gewünschten Wirkstoffmengen pro Flächeneinheit ausgebracht werden. Die Konzentration der Spritzbrühe wird so gewählt, dass in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Die erfindungsgemässen Wirkstoffe zeigen in diesem Test eine sehr gute herbizide Wirksamkeit.

**Patentansprüche**

1. 1-(2-Oxyaminosulfonylphenylsulfonyl)-3-heteroarylharnstoffe der allgemeinen Formel (I)

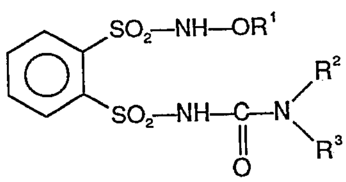

(I)

in welcher $R^1$ für $C_1$-$C_{12}$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_3$-Cycloalkyl, $C_6$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl, oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht, in welcher weiter

$R^2$ für Wasserstoff, $C_1$-$C_4$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl, $C_3$-$C_6$-Alkinyl oder Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist) steht, in welcher ferner

$R^3$ für den Rest

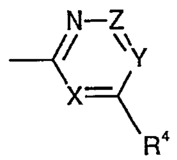

steht, worin

$R^4$ für Wasserstoff, Fluor, Chlor, Brom, Hydroxy, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist, $C_1$-$C_4$-Alkylthio (welches gegebenenfalls durch Fluor und/oder Chlor substituiert ist), Amino, $C_1$-$C_4$-Alkylamino oder Di-($C_1$-$C_4$-alkyl)-amino steht,

X für Stickstoff oder eine Methin-Brücke (CH) steht,

Y für Stickstoff oder eine gegebenenfalls substituierte Methin-brücke C–$R^5$ steht, wobei

$R^5$ für Wasserstoff, Fluor, Chlor, Brom, Cyano, Formyl, $C_1$-$C_4$-Alkyl, $C_1$-$C_3$-Alkoxy-carbonyl oder $C_1$-$C_3$-Alkyl-carbonyl steht, und

Z für Stickstoff oder eine gegebenenfalls substituierte Methin-brücke C-$R^6$ steht, wobei

$R^6$ für Wasserstoff, Fluor, Chlor, Brom, Hydroxy, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio (welches gegebenenfalls durch Fluor und/oder Chlor substituiert ist), Amino, $C_1$-$C_4$-Alkylamino oder Di($C_1$-$C_4$-alkyl)-amino steht,

wobei folgende Verbindungen ausgenommen sind:

1-(2-Methoxyaminosulfonylphenylsulfonyl)-,
1-(2-Ethoxyaminosulfonylphenylsulfonyl)-,
1-(2-Propoxyaminosulfonylphenylsulfonyl)-,
1-(2-Isopropoxyaminosulfonylphenylsulfonyl)-
und
1-(2-Butoxyaminosulfonylphenylsulfonyl)-,
-3-(4,6-dimethyl-pyrimidin-2-yl)-harnstoff,
-3-(4,6-diethyl-pyrimidin-2-yl)-harnstoff,
-3-(4,6-dipropyl-pyrimidin-2-yl)-harnstoff,
-3-(4,6-diisopropyl-pyrimidin-2-yl)-harnstoff und
-3-(4,6-dibutyl-pyrimidin-2-yl)-harnstoff.

2. 1-(2-Oxyaminosulfonylphenylsulfonyl)-3-heteroaryl-harnstoffe der allgemeinen Formel (I) gemäss Anspruch 1, dadurch gekennzeichnet, dass darin

$R^1$ für $C_1$-$C_8$-Alkyl (welches gegebenenfalls durch Fluor oder Chlor substituiert ist), $C_3$-$C_4$-Alkenyl, $C_1$-$C_2$-Alkoxy-carbonylmethyl, Phenyl, Phenylethyl oder Benzyl (welches gegebenenfalls durch Fluor, Chlor, Nitro, Cyano, Methyl, Methoxy oder Methoxy-carbonyl substituiert ist) steht,
$R^2$ für Wasserstoff steht und
$R^3$ für den Rest

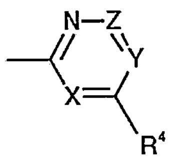

steht, worin

$R^4$ für Chlor, Methyl, Ethyl, Methoxy, Difluormethoxy oder Ethoxy steht,
X für Stickstoff steht,
Y für eine Methin-brücke (CH) steht, und
Z für eine gegebenenfalls substituierte Methinbrücke C-$R^6$ steht, wobei
$R^6$ für Wasserstoff, Chlor, Methyl, Methoxy, Ethoxy, Methylthio, Ethylthio, Dimethylamino oder Diethylamino steht,
wobei folgende Verbindungen ausgenommen sind:
1-(2-Methoxyaminosulfonylphenylsulfonyl)-,
1-(2-Ethoxyaminosulfonylphenylsulfonyl)-,
1-(2-Propoxyaminosulfonylphenylsulfonyl)-,
1-(2-Isopropoxyaminosulfonylphenylsulfonyl)- und
1-(2-Butoxyaminosulfonylphenylsulfonyl)-,
-3-(4,6-dimethyl-pyrimidin-2-yl)-harnstoff,
-3-(4,6-diethyl-pyrimidin-2-yl)-harnstoff,
-3-(4,6-dipropyl-pyrimidin-2-yl)-harnstoff,
-3-(4,6-diisopropyl-pyrimidin-2-yl)-harnstoff und
-3-(4,6-dibutyl-pyrimidin-2-yl)-harnstoff.

3. 1-(2-Methoxyaminosulfonyl-phenylsulfonyl)-3-(4-methoxy-6-methyl-pyrimidin-2-yl)-harnstoff der Formel

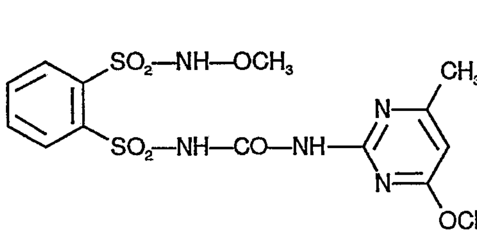
(21)

gemäss Anspruch 1.

4. 1-(2-Ethoxyaminosulfonyl-phenylsulfonyl)-3-(4-methoxy-6-methyl-pyrimidin-2-yl)-harnstoff der Formel

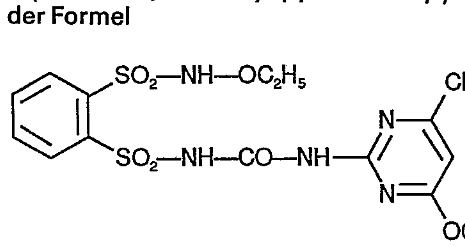
(22)

gemäss Anspruch 1.

5. 1-(Methoxyaminosulfonyl-phenylsulfonyl)-3-(4-ethoxy-6-methyl-pyrimidin-2-yl)-harnstoff der Formel

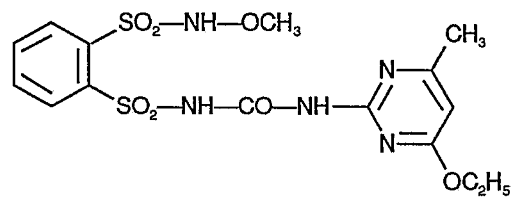
(27)

gemäss Anspruch 1.

6. 1-(2-Methoxyaminosulfonyl-phenylsulfonyl)-3-(4,6-dimethoxy-pyrimidin-2-yl)-harnstoff der Formel

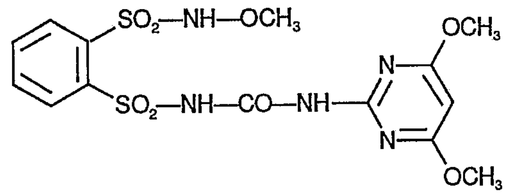
(34)

gemäss Anspruch 1.

7. Verfahren zur Herstellung von 1-(2-Oxyaminosulfonyl-phenylsulfonyl)-3-heteroaryl-harnstoffen der allgemeinen Formel (I) gemäss Anspruch 1, dadurch gekennzeichnet, dass man
(a) Benzodisultame der Formel (II)

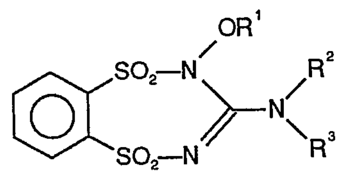
(II)

in welcher
$R^1$, $R^2$ und $R^3$ die in Anspruch 1 angegebenen Bedeutungen haben,
mit Wasser gegebenenfalls in Gegenwart von Basen und gegebenenfalls in Gegenwart von Verdünnungsmitteln umsetzt, oder
(b) Benzol-1,2-disulfonsäure-dichlorid der Formel (III)

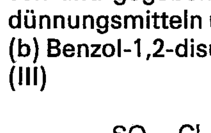
(III)

mit Oxyguanidin-Derivaten der Formel (IV)

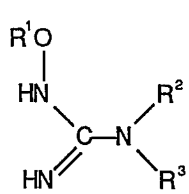
(IV)

in welcher
$R^1$, $R^2$ und $R^3$ die in Anspruch 1 angegebene Bedeutung haben, in Gegenwart von Säureakzeptoren und gegebenenfalls in Gegenwart von Verdünnungsmitteln umsetzt und die hierbei erhaltenen Verbindungen der Formel (II) – ohne Zwischenisolierung – mit Wasser gegebenenfalls in Gegenwart von Basen und gegebenenfalls in Gegenwart von Verdünnungsmitteln umsetzt.

8. Verfahren nach Anspruch 7(b), dadurch gekennzeichnet, dass man Benzol-1,2-disulfonsäure-dichlorid der Formel (III) mit N′-(4-Methoxy-6-methyl-pyrimidin-2-yl)-N″-methoxy-guanidin der Formel (IV-44)

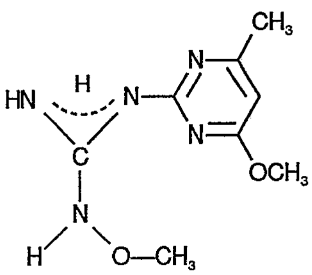

(IV–44)

umsetzt.

9. Verfahren nach Anspruch 7(b), dadurch gekennzeichnet, dass man Benzol-1,2-disulfonsäure-dichlorid der Formel (III) mit N′-(4,6-Dimethoxy-pyrimidin-2-yl)-N″-methoxy-guanidin der Formel (IV-52)

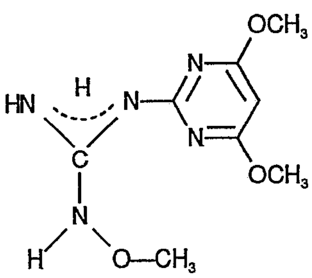

(IV–52)

umsetzt.

10. Herbizide Mittel, gekennzeichnet durch einen Gehalt an mindestens einem 1-(2-Oxyaminosulfonylphenylsulfonyl)-3-heteroaryl-harnstoff der allgemeinen Formel (I) gemäss Anspruch 1.

11. Verwendung von 1-(2-Oxyaminosulfonylphenylsulfonyl)-3-heteroaryl-harnstoffen der allgemeinen Formel (I) gemäss Anspruch 1 zur Bekämpfung von unerwünschtem Pflanzenwachstum.

12. Verfahren zur Herstellung von herbiziden Mitteln, dadurch gekennzeichnet, dass man 1-(2-Oxyaminosulfonylphenylsulfonyl)-3-heteroaryl-harnstoffe der allgemeinen Formel (I) gemäss Anspruch 1 mit Streckmitteln und/oder oberflächenaktiven Mitteln vermischt.

## Claims

1. 1-(2-Oxyaminosulfonylphenylsulfonyl)-3-heteroaryl-ureas of the general formula (I)

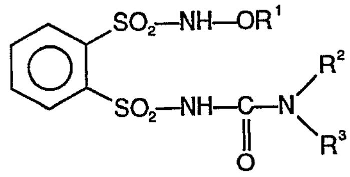

(I)

in which
$R^1$ represents $C_1$-$C_{12}$-alkyl (which is optionally substituted by fluorine, chlorine, cyano, $C_1$-$C_4$-alkoxy, $C_1$-$C_4$-alkylthio, $C_1$-$C_4$-alkylsulphinyl, $C_1$-$C_4$-alkylsulphonyl, $C_1$-$C_4$-alkyl-carbonyl, $C_1$-$C_4$-alkoxy-carbonyl, $C_1$-$C_4$-alkylamino-carbonyl or di-($C_1$-$C_4$-alkyl)-amino-carbonyl), or represents $C_3$-$C_6$-alkenyl (which is optionally substituted by fluorine, chlorine or bromine), $C_3$-$C_6$-alkinyl, $C_3$-$C_6$-cycloalkyl, $C_3$-$C_6$-cycloalkyl-$C_1$-$C_2$-alkyl or phenyl-$C_1$-$C_2$-alkyl (which is optionally substituted by fluorine, chlorine, nitro, cyano, $C_1$-$C_4$-alkyl, $C_1$-$C_4$-alkoxy or $C_1$-$C_4$-alkoxy-carbonyl), or represents benzhydryl, or represents phenyl (which is optionally substituted by fluorine, chlorine, nitro, cyano, $C_1$-$C_4$-alkyl, trifluoromethyl, $C_1$-$C_4$-alkoxy, $C_1$-$C_2$-fluoroalkoxy, $C_1$-$C_4$-alkylthio, trifluoromethylthio or $C_1$-$C_4$-alkoxy-carbonyl), and in which, furthermore, $R^2$ represents hydrogen or $C_1$-$C_4$-alkyl (which is optionally substituted by fluorine, chlorine, cyano, $C_1$-$C_4$-alkoxy, $C_1$-$C_4$-alkylthio, $C_1$-$C_4$-alkylsulphinyl, $C_1$-$C_4$-alkylsulphonyl, $C_1$-$C_4$-alkyl-carbonyl, $C_1$-$C_4$-alkoxy-carbonyl, $C_1$-$C_4$-alkylamino-carbonyl or di-($C_1$-$C_4$-alkyl)-amino-carbonyl), or represents $C_3$-$C_6$-alkenyl, $C_3$-$C_6$-alkinyl or phenyl-$C_1$-$C_2$-alkyl (which is optionally substituted by fluorine, chlorine, nitro, cyano, $C_1$-$C_4$-alkyl, $C_1$-$C_4$-alkoxy or $C_1$-$C_4$-alkoxy-carbonyl), and in which, furthermore,
$R^3$ represents the radical

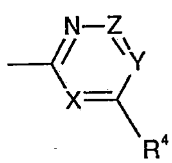

wherein
$R^4$ represents hydrogen, fluorine, chlorine, bromine, hydroxyl, $C_1$-$C_4$-alkyl (which is optionally substituted by fluorine and/or chlorine), $C_1$-$C_4$-alkoxy (which is optionally substituted by fluorine and/or chlorine), $C_1$-$C_4$-alkylthio (which is optionally substituted by fluorine and/or chlorine), amino, $C_1$-$C_4$-alkylamino or di-($C_1$-$C_4$-alkyl)-amino, X represents nitrogen or a methine bridge (CH), Y represents nitrogen or an optionally substituted methine bridge C-$R^5$,
wherein
$R^5$ represents hydrogen, fluorine, chlorine, bromine, cyano, formyl, $C_1$-$C_4$-alkyl, $C_1$-$C_3$-alkoxy-carbonyl or $C_1$-$C_3$-alkyl-carbonyl, and Z represents nitrogen or an optionally substituted methine bridge C-$R^6$,
wherein
$R^6$ represents hydrogen, fluorine, chlorine, bromine, hydroxyl, $C_1$-$C_4$-alkyl (which is optionally substituted by fluorine and/or chlorine), $C_1$-$C_4$-alkoxy (which is optionally substituted by fluorine

and/or chlorine), $C_1$-$C_4$-alkylthio (which is optionally substituted by fluorine and/or chlorine), amino, $C_1$-$C_4$-alkylamino or di-($C_1$-$C_4$-alkyl)-amino, the following compounds being excluded:

1-(2-methoxyaminosulfonylphenylsulfonyl)-, 1-(2-ethoxyaminosulfonylphenylsulfonyl)-, 1-(2-propoxyaminosulfonylphenylsulfonyl)-, 1-(2-isopropoxyaminosulfonylphenylsulfonyl)- and 1-(2-butoxyaminosulfonylphenylsulfonyl)-3-(4,6-dimethyl-pyrimidin-2-yl)-urea, -3-(4,6-diethyl-pyrimidin-2-yl)-urea, -3-(4,6-dipropyl-pyrimidin-2-yl)-urea, -3-(4,6-diisopropyl-pyrimidin-2-yl)-urea and -3-(4,6-dibutyl-pyrimidin-2-yl)-urea.

2. 1-(2-Oxyaminosulfonylphenylsulfonyl)-3-heteroarylureas of the general formula (I) according to Claim 1, characterized in that
$R^1$ represents $C_1$-$C_8$-alkyl (which is optionally substituted by fluorine or chlorine), $C_3$-$C_4$-alkenyl, $C_1$-$C_2$-alkoxy-carbonylmethyl, phenyl, phenethyl or benzyl (which is optionally substituted by fluorine, chlorine, nitro, cyano, methyl, methoxy or methoxy-carbonyl),
$R^2$ represents hydrogen and
$R^3$ represents the radical

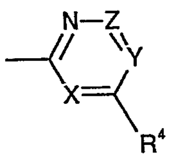

wherein
$R^4$ represents chlorine, methyl, ethyl, methoxy, difluoromethoxy or ethoxy, X represents nitrogen, Y represents a methine bridge (CH) and Z represents an optionally substituted methine bridge C-$R^6$,
wherein
$R^6$ represents hydrogen, chlorine, methyl, methoxy, ethoxy, methylthio, ethylthio, dimethylamino or diethylamino, the following compounds being excluded:

1-(2-methoxyaminosulfonylphenylsulfonyl)-, 1-(2-ethoxyaminosulfonylphenylsulfonyl)-, 1-(2-propoxyaminosulfonylphenylsulfonyl)-, 1-(2-isopropoxyaminosulfonylphenylsulfonyl)- and 1-(2-butoxyaminosulfonylphenylsulfonyl)-3-(4,6-dimethyl-pyrimidin-2-yl)-urea, -3-(4,6-diethyl-pyrimidin-2-yl)-urea, -3-(4,6-dipropyl-pyrimidin-2-yl)-urea, -3-(4,6-diisopropyl-pyrimidin-2-yl)-urea and -3-(4,6-dibutyl-pyrimidin-2-yl)-urea.

3. 1-(2-methoxyaminosulfonyl-phenylsulfonyl)-3-(4-methoxy-6-methyl-pyrimidin-2-yl)-urea of the formula

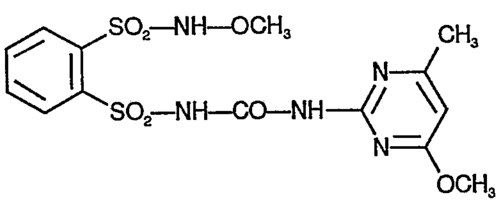
(21)

according to Claim 1.

4. 1-(2-Ethoxyaminosulfonyl-phenylsulfonyl)-3-(4-methoxy-6-methyl-pyrimidin-2-yl)-urea of the formula

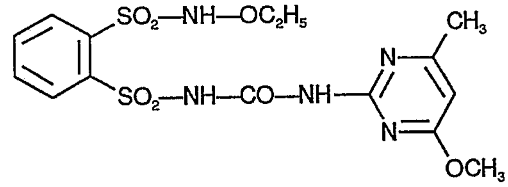
(22)

according to Claim 1.

5. 1-(Methoxyaminosulfonyl-phenylsulfonyl)-3-(4-ethoxy-6-methyl-pyrimidin-2-yl)-urea of the formula

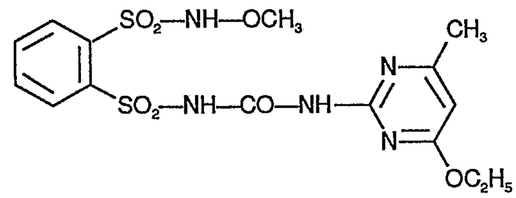
(27)

according to Claim 1.

6. 1-(2-methoxyaminosulfonyl-phenylsulfonyl)-3-(4,6-dimethoxy-pyrimidin-2-yl)-urea of the formula

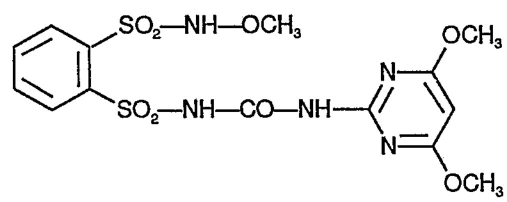
(34)

according to Claim 1.

7. Process for the preparation of 1-(2-oxyaminosulfonylphenylsulfonyl)-3-heteroaryl-ureas of the general formula (I) according to Claim 1, characterized in that
(a) benzodisultams of the formula (II)

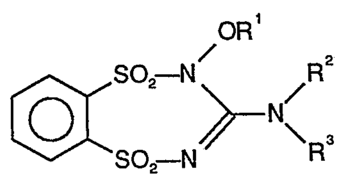
(II)

in which
R, $R^2$ and $R^3$ have the meanings given in Claim 1, are reacted with water, if appropriate in the presence of bases and if appropriate in the presence of diluents, or (b) benzene-1,2-disulphonic acid dichloride of the formula (III)

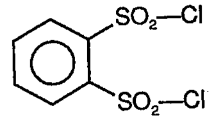
(III)

is reacted with oxyguanidine derivatives of the formula (IV)

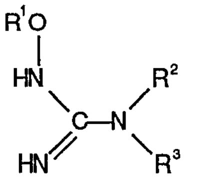

(IV)

in which
$R^1$, $R^2$ and $R^3$ have the meanings given in Claim 1, in the presence of acid acceptors and if appropriate in the presence of diluents, and the compounds of the formula (II) thereby obtained are reacted - without intermediate isolation - with water, if appropriate in the presence of bases and if appropriate in the presence of diluents.

8. Process according to Claim 7(b), characterized in that benzene-1,2-disulphonic acid dichloride of the formula (III) is reacted with N′-(4-methoxy-6-methyl-pyrimidin-2-yl)-N″-methoxy-guanidine of the formula (IV-44)

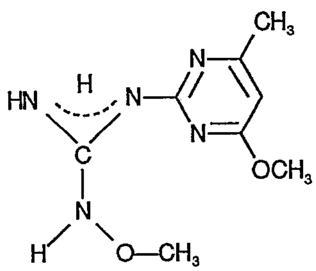

(IV–44)

9. Process according to Claim 7(b), characterised in that benzene-1,2-disulphonic acid dichloride of the formula (III) is reacted with N′(4,6-dimethoxy-pyrimidin-2-yl)-N″-methoxy-guanidine of the formula (IV-52)

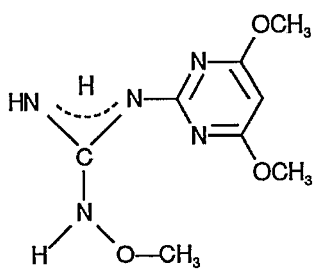

(IV–52)

10. Herbicidal agents, characterized in that they contain at least one 1-(2-oxyaminosulphonylphenylsulphonyl)-3-heteroaryl-urea of the general formula (I) according to Claim 1.

11. Use of 1-(2-oxyaminosulphonylphenylsulphonyl)-3-heteroaryl-ureas of the general formula (I) according to Claim 1 for combating undesirable plant growth.

12. Process for the preparation of herbicidal agents, characterized in that 1-(2-oxyaminosulphonylphenyl-sulphonyl)-3-heteroaryl-ureas of the general formula (I) according to Claim 1 are mixed with extenders and/or surface-active agents.

**Revendications**

1. 1-(2-oxyaminosulfonylphénylsulfonyl)-3-hétéroarylurées de formule générale (I)

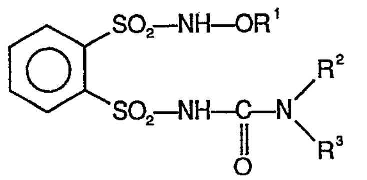

(I)

dans laquelle
$R^1$ représente un groupe alkyle en $C_1$ à $C_{12}$ [qui est éventuellement substitué par du fluor, du chlore, un radical cyano, alkoxy en $C_1$ à $C_4$, alkylthio en $C_1$ à $C_4$, alkylsulfinyle en $C_1$ à $C_4$, alkylsulfonyle en $C_1$ à $C_4$, (alkyle en $C_1$ à $C_4$)carbonyle, (alkoxy en $C_1$ à $C_4$)carbonyle, (alkyle en $C_1$ à $C_4$)amino-carbonyle ou di(alkyle en $C_1$ à $C_4$) amino-carbonyle], un groupe alcényle en $C_3$ à $C_6$ (qui est substitué le cas échéant par du fluor, du chlore ou du brome), un groupe alcynyle en $C_3$ à $C_6$, cycloalkyle en $C_3$ à $C_6$, (cycloalkyle en $C_3$ à $C_6$-alkyle en $C_1$ à $C_2$, phényl-(alkyle en $C_1$ à $C_2$) [qui est éventuellement substitué par du fluor, du chlore, un radical nitro, cyano, alkyle en $C_1$ à $C_4$, alkoxy en $C_1$ à $C_4$ ou (alkoxy en $C_1$ à $C_4$)carbonyle], un groupe benzhydryle ou un groupe phényle [qui est éventuellement substitué par du fluor, du chlore, un radical nitro, cyano, alkyle en $C_1$ à $C_4$, trifluorométhyle, alkoxy en $C_1$ à $C_4$, fluoralkoxy en $C_1$ ou $C_2$, alkylthio en $C_1$ à $C_4$, trifluorméthylthio ou (alkoxy en $C_1$ à $C_4$)carbonyle], dans laquelle en outre représente l'hydrogène, un groupe alkyle en $C_1$ à $C_4$ [qui est éventuellement substitué par du fluor, du chlore, un radical cyano, alkoxy en $C_1$ à $C_4$, alkylthio en $C_1$ à $C_4$, alkylsulfinyle en $C_1$ à $C_4$, alkylsulfonyle en $C_1$ à $C_4$, (alkyle en $C_1$ à $C_4$)-carbonyle, (alkoxy en $C_1$ à $C_4$)-carbonyle, (alkyle en $C_1$ à $C_4$)amino-carbonyle ou di(alkyle en $C_1$ à $C_4$ amino-carbonyle], un groupe alcényle en $C_3$ à $C_6$, alcynyle en $C_3$ à $C_6$ ou phényl-(alkyle en $C_1$ ou $C_2$), [qui est éventuellement substitué par du fluor, du chlore, un radical nitro, cyano, alkyle en $C_1$ à $C_4$, alkoxy en $C_1$ à $C_4$ ou (alkoxy en $C_1$ à $C_4$)-carbonyle], dans laquelle en outre $R^3$ représente le reste

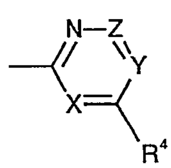

dans lequel
$R^4$ représente l'hydrogène, le fluor, le chlore, le brome, un groupe hydroxy, alkyle en $C_1$ à $C_4$ [qui est éventuellement substitué par du fluor et/ou du chlore], un groupe alkoxy en $C_1$ à $C_4$ [qui est éventuellement substitué par du fluor et/ou du chlore], un groupe alkylthio en $C_1$ à $C_4$ [qui est éventuellement substitué par du fluor et/ou du chlore], un groupe amino, alkylamino en $C_1$ à $C_4$ ou di(alkyle en $C_1$ à $C_4$-amino,
X représente l'azote ou un pont méthine (CH),

Y représente l'azote ou un pont méthine C-$R^5$ éventuellement substitué, dans lequel
$R^5$ représente l'hydrogène, le fluor, le chlore, le brome, un groupe cyano, formyle, alkyle en $C_1$ à $C_4$, (alkoxy en $C_1$ à $C_3$)-carbonyle ou alkyle en $C_1$ à $C_3$-carbonyle, et
Z représente l'azote ou un pont méthine C-$R^6$ éventuellement substitué, dans lequel $R^6$ représente l'hydrogène, le fluor, le chlore, le brome, un groupe hydroxy, alkyle en $C_1$ à $C_4$ [qui est éventuellement substitué par du fluor et/ou du chlore], un groupe alkoxy en $C_1$ à $C_4$ [qui est éventuellement substitué par du fluor et/ou du chlore], un groupe alkylthio en $C_1$ à $C_4$ [qui est éventuellement substitué par du fluor et/ou du chlore], un groupe amino, alkylamino en $C_1$ à $C_4$ ou di(alkyle en $C_1$ à $C_4$)-amino,
à l'exclusion des composés suivants:

1-(2-méthoxyaminosulfonylphénylsulfonyl)-,
1-(2-éthoxyaminosulfonylphénylsulfonyl)-,
1-(2-propoxyaminosulfonylphénylsulfonyl)-,
1-(2-isopropoxyaminosulfonylphénylsulfonyl)- et
1-(2-butoxyaminosulfonylphénylsulfonyl)-,
3-(4,6-diméthyl-pyrimidine-2-yl)-urée,
-3-(4,6-diéthyl-pyrimidine-2-yl)-urée,
-3-(4,6-dipropyl-pyrimidine-2-yl)-urée,
-3-(4,6-diisopropyl-pyrimidine-2-yl)-urée et
-3-(4,6-dibutyl-pyrimidine-2-yl)-urée.

2. 1-(2-oxyaminosulfonylphénylsulfonyl)-3-hétéroarylurées de formule générale (I) suivant la revendication 1, caractérisées en ce que, dans cette formule
$R^1$ représente un groupe alkyle en $C_1$ à $C_8$ [qui est éventuellement substitué par du fluor ou du chlore], un groupe alcényle en $C_3$ ou $C_4$, (alkoxy en $C_1$ ou $C_2$)-carbonylméthyle, phényle, phényléthyle ou benzyle [qui est éventuellement substitué par du fluor, du chlore, un radical nitro, cyano, méthyle, méthoxy ou méthoxycarbonyl],
$R^2$ représente l'hydrogène et
$R^3$ est le reste

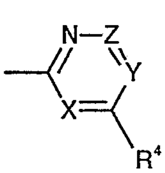

dans lequel
$R^4$ représente le chlore, un groupe méthyle, éthyle, méthoxy, difluorométhoxy ou éthoxy,
X représente l'azote,
Y est un pont méthine (CH) et
Z est un pont méthine C-$R^6$ éventuellement substitué, dans lequel
$R^6$ représente l'hydrogène, le chlore, un groupe méthyle, méthoxy, éthoxy, méthylthio, éthylthio, diméthylamino ou diéthylamino,
les composés suivants étant exclus:
1-(2-méthoxyaminosulfonylphénylsulfonyl)-,
1-(2-éthoxyaminosulfonylphénylsulfonyl)-,
1-(2-propoxyaminosulfonylphénylsulfonyl)-,
1-(2-isopropoxyaminosulfonylphénylsulfonyl)- et
1-(2-butoxyaminosulfonylphénylsulfonyl)-,
3-(4,6-diméthyl-pyrimidine-2-yl)-urée,
-3-(4,6-diéthyl-pyrimidine-2-yl)-urée,
-3-(4,6-dipropyl-pyrimidine-2-yl)-urée,
-3-(4,6-diisopropyl-pyrimidine-2-yl)-urée, et
-3-(4,6-dibutyl-pyrimidine-2-yl)-urée.

3. La 1-(2-méthoxyaminosulfonylphénylsulfonyl)-3-(4,6-méthoxy-6-méthylpyrimidine-2-yl)-urée de formule

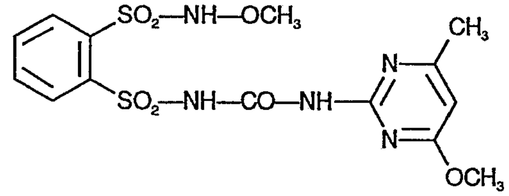

(21)

suivant la revendication 1.

4. La 1-(2-éthoxyaminosulfonyl-phénylsulfonyl)-3-(4-méthoxy-6-méthylpyrimidine-1-yl)-urée de formule

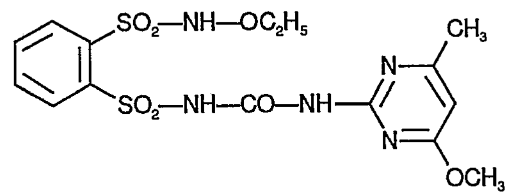

(22)

suivant la revendication 1.

5. La 1-(méthoxyaminosulfonyl-phénylsulfonyl)-3-(4-éthoxy-6-méthylpyrimidine-2-yl)-urée de formule

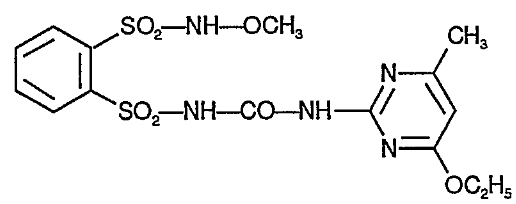

(27)

suivant la revendication 1.

6. La 1-(2-méthoxyaminosulfonyl-phénylsulfonyl)-3-(4,6-diméthoxypyrimidine-2-yl)-urée de formule

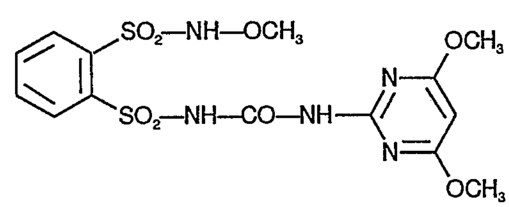

(34)

suivant la revendication 1.

7. Procédé de production de 1-(2-oxyaminosulfonylphénylsulfonyl)-3-hétéroaryl-urée de formule générale (I) suivant la revendication 1, caractérisé en ce que
(a) on fait réagir des benzodisultames de formule (II)

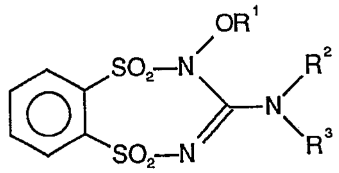

(II)

dans laquelle
$R^1$, $R^2$ et $R^3$ ont les définitions indiquées dans la revendication 1, avec l'eau, éventuellement en présence de bases et le cas échéant en présence de diluants, ou bien
(b) on fait réagir le dichlorure d'acide benzène-1,2-disulfonique de formule (III)

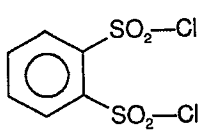

(III)

avec des dérivés d'oxyguanidine de formule (IV)

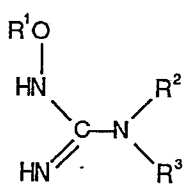

(IV)

dans laquelle
$R^1$, $R^2$ et $R^3$ ont la définition indiquée dans la revendication 1, en présence d'accepteurs d'acides et, le cas échéant, en présence de diluants, et on fait réagir les composés de formule (II) ainsi obtenus - sans isolement intermédiaire - avec l'eau, le cas échéant en présence de bases et en la présence éventuelle de diluants.

8. Procédé suivant la revendication 7 (b), caractérisé en ce qu'on fait réagir le dichlorure d'acide benzène-1,2-disulfonique de formule (III) avec la N'-(4-méthoxy-6-méthyl-pyrimidine-2-yl)-N″-méthoxy-guanidine de formule (IV-44)

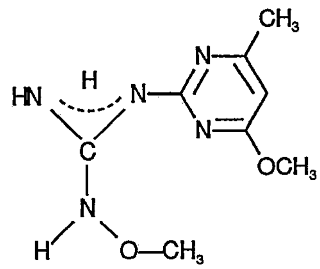

(IV-44)

9. Procédé suivant la revendication 7(b), caractérisé en ce qu'on fait réagir le dichlorure d'acide benzène-1,2-disulfonique de formule (III) avec la N'-(4,6-diméthoxy-pyrimidine-2-yl)-N″-méthoxy-guanidine de formule (IV-52)

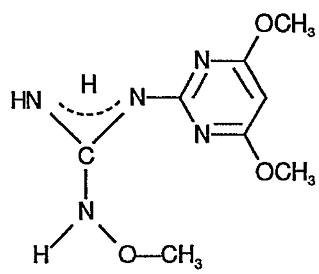

(IV-52)

10. Compositions herbicides, caractérisées par une teneur en au moins une 1-(2-oxyaminosulfonylphénylsulfonyl)-3-hétéroarylurée de formule générale (I) suivant la revendication 1.

11. Utilisation de 1-(2-oxyaminosulfonylphénylsulfonyl)-3-hétéroarylurées de formule générale (I) suivant la revendication 1 pour combattre la croissance non désirée de plantes.

12. Procédé de préparation de compositions herbicides, caractérisé en ce qu'on mélange des 1-(2-oxyaminosulfonylphénylsulfonyl)-3-hétéroarylurées de formule générale (I) suivant la revendication 1 avec des diluants et/ou des agents tensio-actifs.